# EUROPÄISCHE PATENTSCHRIFT

(11) **EP 0 510 449 B1**
(45) Veröffentlichungstag und Bekanntmachung des Hinweises auf die Patenterteilung: **05.08.1998**
(21) Anmeldenummer: 92106145.3
(22) Anmeldetag: 09.04.1992
(51) Int. Cl.: C08G 71/00, C08G 69/00, C07C 271/12, C07C 271/14, C07C 271/16, C07C 275/06, C07C 235/00, G03F 7/039

(54) **Durch Säure spaltbare Verbindungen, diese enthaltende positiv strahlungsempfindliche Zusammensetzung und damit hergestelltes positiv strahlungsempfindliches Aufzeichnungsmaterial**
Acid-cleavable compounds, positive-working radiation-sensitive composition containing the same and radiation-sensitive recording material prepared therefrom
Composés à clivage par un acide, composition radiosensible positive contenant les mêmes et matériau d'enregistrement sensible préparé de ceux-ci

(30) Priorität: 20.04.1991 DE 4112968
(43) Veröffentlichungstag der Anmeldung: 28.10.1992
(73) Patentinhaber: Clariant GmbH, 65929 Frankfurt am Main (DE)
(72) Erfinder: Pawlowski, Georg, Dr., W-6200 Wiesbaden (DE); Röschert, Horst, Dr., W-6531 Ober Hilbersheim (DE); Spiess, Walter, Dr., W-6110 Dieburg (DE); Dammel, Ralph, Dr., Coventry, Rhode Island 02816 (US)

(56) Entgegenhaltungen:
- EP-A- 0 417 556
- EP-A- 0 424 766
- US-A- 3 779 778
- US-A- 3 981 913
- US-A- 3 996 269
- US-A- 4 031 271
- US-A- 4 101 323
- POLYMER ENGINEERING AND SCIENCE, Bd.23, Nr.17, Dezember 1983 Seiten 953 - 956 J.V.CRIVELLO 'possibilities for photoimaging using onium salts'

## Beschreibung

Die Erfindung betrifft neue säurespaltbare N,O-Acetale sowie ein positiv arbeitendes, d.h. durch Bestrahlung löslich werdendes strahlungsempfindliches Gemisch, das diese Verbindungen enthält. Das Gemisch enthält allgemein
(a) ein in Wasser unlösliches, in wäßrig-alkalischen Lösungen lösliches oder zumindest quellbares Bindemittel,
(b) eine Verbindung, die unter Einwirkung von aktinischer Strahlung eine starke Säure bildet, sowie
(c) eine Verbindung, die mindestens eine durch Säure spaltbare C-O-C-Bindung aufweist und sich durch die Spaltung in Produkte mit erhöhter Löslichkeit in wäßrig-alkalischen Lösungen umwandeln läßt.

Die Erfindung betrifft weiterhin ein damit hergestelltes strahlungsempfindliches Aufzeichnungsmaterial, das zur Herstellung von Photoresists, elektronischen Bauteilen, Druckplatten oder zum Formteilätzen geeignet ist.

Zur Herstellung mikroelektronischer Schaltungen finden derzeit mehrere lithographische Techniken Anwendung. Mit der g-line-Lithographie (436 nm), die üblicherweise auf konventionelle Diazonaphthochinon/ Novolak-Formulierungen angewendet wird, können Resistbilder mit einer Auflösung von bis zu 0,8 µm hergestellt werden. Noch feinere Strukturen (bis herab zu 0,5 µm) lassen sich mit Hilfe der I-Line-Lithographie (365 nm) auf einer Resistschicht abbilden. Neuere Modifikationen der i-line-Lithographie, beispielsweise die Phase-Shifting-Mask-Technologie, erlauben eine weitere Verkleinerung der abzubildenden Strukturen bis herab zu etwa 0,35 µm oder kleiner. Eine noch höhere Auflösung läßt sich mit UV2-Photoresists erreichen. Dabei werden zwei Bestrahlungstechniken eingesetzt: die UV2-Breitbandbelichtung (240 bis 260 nm) oder die Belichtung mit KrF-Excimer-Lasern, die bei 248 nm emittieren.

Die stetige Verkleinerung der Strukturen, beispielsweise in der Chip-Herstellung bis in den Bereich von weniger als 1 µm, erfordert veränderte lithographische Techniken. Um solche feinen Strukturen abzubilden, verwendet man Strahlung mit einer kurzen Wellenlänge, wie energiereiches UV-Licht, Elektronen- und Röntgenstrahlen. Das strahlungsempfindliche Gemisch muß an die kurzwellige Strahlung angepaßt sein. Eine Zusammenstellung der an das strahlungsempfindliche Gemisch gestellten Forderungen ist in dem Aufsatz von C.G. Willson "Organic Resist Materials - Theory and Chemistry" [Introduction to Microlithography, Theory, Materials, and Processing, Herausgeber L.F. Thompson, C.G. Willson, M.J. Bowden, ACS Symp. Ser., 219, 87 (1983), American Chemical Society, Washington] angegeben. Es bestand daher ein verstärkter Bedarf an strahlungsempfindlichen Gemischen, die in den neueren Technologien, wie der Mid- oder Deep-UV-Lithographie, [Belichtung z. B. mit Excimer-Lasern bei Wellenlängen von 305 nm (XeF), 248 nm (KrF), 193 nm (ArF)], der Elektronen- oder der Röntgenstrahl-Lithographie, einsetzbar sind, vorzugsweise darüber hinaus in einem weiten Spektralbereich empfindlich sind und dementsprechend auch in der konventionellen UV-Lithographie verwendet werden können.

Positiv arbeitende strahlungsempfindliche Gemische zur Herstellung strahlungsempfindlicher Aufzeichnungsmaterialien sind bekannt. Handelsüblich sind Gemische, die o-Chinon-diazid-Derivate in wäßrig-alkalisch löslichen Bindemitteln, beispielsweise Novolaken oder Polyhydroxystyrolen, enthalten. Die Empfindlichkeit dieser Materialien gegenüber aktinischer, insbesondere aber hochenergetischer, kurzwelliger Strahlung, wie Licht eines KrF-Excimer-Lasers mit einer Wellenlänge von 248 nm oder Elektronenstrahlung, ist aber unzureichend.

Weiterhin sind positiv arbeitende strahlungsempfindliche Gemische bekannt, bei denen durch Einwirkung von aktinischer Strahlung auf einen in dieser Mischung enthaltenen Photoinitiator eine Säure gebildet wird und diese dann in einer Folgereaktion eine ebenfalls in der Mischung enthaltene säurespaltbare Verbindung in den bestrahlten Bereichen gegenüber einem entsprechenden, bevorzugt wäßrig-alkalischen Entwickler löslich macht. Derartige Materialien zeichnen sich im allgemeinen durch eine verbesserte Empfindlichkeit gegenüber aktinischer Strah-lung aus.

Es sind zahlreiche Gemische aus säurespaltbaren, löslichkeitsinhibierenden Verbindungen bekannt, die immer eine geringe Menge eines photolytischen Säurebildners enthalten, dessen saures Photolyseprodukt die Spaltung initiiert. Diese Gemische mit z.T. hohen Empfindlichkeiten gegenüber aktinischer Strah-lung werden als chemisch verstärkte, photokatalytische 3-Komponentensysteme bezeichnet, da sie als wesentliche Bestandteile ein polymeres, in wäßrig-alkalischen Lösungen lösliches Bindemittel, zumeist ein Novolak-Harz, eine photoaktive Verbindung und einen Löslichkeitsinhibitor enthalten.

Unter diesen Gemischen haben insbesondere solche eine gewisse kommerzielle Bedeutung erlangt, die als säurespaltbare Komponenten entweder monomere oder oligomere Acetaleinheiten aufweisen. Diese Gemische haben jedoch gewisse Nachteile, denn sie besitzen auf den Substratmaterialien, auf die sie aufgebracht werden müssen, nur eine begrenzte Stabilität und führen zu unzureichenden, nicht reproduzierbaren Vorlagewiedergaben, was nur durch Einführung zusätzlicher Schutzschichten, z. B. gemäß DE-A 36 21 376 (= US-A 4 840 867), verbessert werden kann. Darüber hinaus ist allgemein beobachtbar, daß das Prozeßfenster, d. h. der Verarbeitungsspielraum, für die Belichtung dieser Gemische sehr schmal und häufig nicht eindeutig reproduzierbar ist, was sich wiederum in unzureichenden Vorlagewiedergaben äußert. Insbesondere hängt die Qualität der Bildwiedergabe stark von der Zeitdifferenz zwischen Belichtung und Entwicklung, der sogenannten "delay time" ab. Die Ursachen der Verschlechterung der Bildwiedergabe sind nicht im einzelnen bekannt oder nur unzureichend untersucht worden. Grundsätzlich ist davon auszugehen, daß Diffusionsvorgänge, die zu diesem Verhalten führen, nicht einfach steuerbar sind. Es läßt sich jedoch zusätzlich vermuten, daß es während der Trocknung des Gemischs auf einem Substratmaterial zu einer partiellen Verdampfung des Photoinitiators oder der säurelabilen Verbindung oder zu einer Entmischung der einzelnen Gemischbestandteile kommt, was besonders häufig bei säurelabilen Verbindungen mit geringer Löslichkeit in den üblichen Beschichtungslösemitteln beobachtet wird.

Der entscheidende Nachteil der bekannten, Acetalgruppen aufweisenden Verbindungen beruht aber darin, daß die durch ihre Spaltung resultierende Löslichkeitsdifferenzierung zwischen belichteten und unbelichteten Bildbereichen im allgemeinen unzureichend ist. Die Ursache hierfür ist darin zu suchen, daß entweder das als Lösungsinhibitor eingesetzte Acetalderivat eine unzureichende Inhibierungseigenschaft aufweist und bei einer bildmäßigen Differenzierung neben den belichteten Bildstellen auch solche stark angegriffen und abgetragen werden, die nicht belichtet wurden, oder aber, daß die belichteten Bereiche keine ausreichende Löslichkeit aufweisen, um bei der Entwicklung eine bildmäßige Differenzierung zu erlauben. Das Problem läßt sich derart zusammenfassen, daß es mit den bekannten Verbindungen nicht gelingt, ein Material bereitzustellen, das eine ausreichend große Löslichkeitsdifferenz zwischen belichteten und unbelichteten Bereichen hervorruft. Während dieser Effekt bei den gemäß dem Stand der Technik verwendeten Novolakharzen im allgemeinen noch akzeptabel ist, beobachtet man bei Verwendung von anderen Polymeren, daß die bekannten Acetalderivate praktisch keinerlei Inhibierungswirkung mehr zeigen und damit eine praxisgerechte Bilddifferenzierung nicht mehr erlauben.

Es besteht daher ein Bedarf an strahlungsempfindlichen Gemischen, die die oben beschriebenen Nachteile nicht aufweisen, eine praxisgerechte Reaktivität besitzen und insbesondere in Kombination mit den unterschiedlichsten Matrixpolymeren gute bildmäßige Differenzierungen erlauben.

Aufgabe der Erfindung war es, neue säurespaltbare Verbindungen sowie ein strahlungsempfindliches Gemisch der eingangs beschriebenen Gattung vorzuschlagen, das diese Verbindungen enthält. Die neuen säurespaltbaren Verbindungen c) sollten einerseits ausreichende Reaktivität, anderseits aber praxisgerechte Stabilität aufweisen. Darüber hinaus sollten sie homogene Mischungen mit den unterschiedlichsten Matrixpolymeren ausbilden und in der unbestrahlten Form als effizienter Lösungsinhibitor wirken, während ihre Spaltprodukte die Entwickelbarkeit der belichteten Bereiche in wäßrig-alkalischen Entwicklern unterstützen sollten. Das die säurespaltbaren Verbindungen enthaltende Gemisch sollte weiterhin sowohl in Lösung als auch auf den in der Praxis verwendeten Substraten möglichst stabil sein und eine Differenzierung zwischen belichteten und unbelichteten Bereichen erlauben, die höchste Auflösungen mit praktisch senkrechten Flanken erlaubt. Darüber hinaus sollte ein strahlungsempfindliches Gemisch bereitgestellt werden, das insbesondere eine Entmischung der Verbindung (b) und der löslichkeitsdifferenzierenden Verbindung (c) weitgehend vermeidet und dadurch ebenfalls eine verbesserte Differenzierung zwischen Bild- und Nichtbildbereichen erlaubt.

Erfindungsgemäß werden säurespaltbare Verbindungen der allgemeinen Formel I 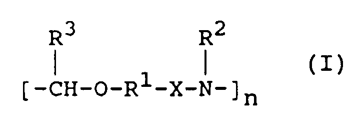 vorgeschlagen, worin
- R¹: eine Alkylen-, Cycloalkylen-, Alkenylen-, Alkinylen- oder Arylenbisalkylgruppe, in der eine oder mehrere aliphatische CH₂-Gruppe(n) durch Sauerstoff- oder Schwefelatome ersetzt sein können,
- R²: einen Alkyl-, Alkenyl-, Alkinyl-, Cycloalkyl-, Alkoxyalkyl-, Aryl-, Aralkyl- oder Aryloxyalkylrest,
- R³: einen Alkyl- oder Arylrest,
- X: eine der Gruppen -CO-, -O-CO- oder -NH-CO- und
- n: eine ganze Zahl größer als 1
bedeuten.

Bevorzugt sind solche Verbindungen der allgemeinen Formel I in denen
- R¹: eine Alkylen-, Alkenylen- oder Alkinylengruppe mit 2 bis 18 Kohlenstoffatomen, in der 1 bis 3 CH₂-Gruppen durch Sauerstoff- oder Schwefelatome ersetzt sein können, eine Cycloalkylengruppe mit 4 bis 18 Kohlenstoffatomen, eine Aralkylengruppe mit 7 bis 18 Kohlenstoffatomen oder eine Arylenbisalkylgruppe mit 8 bis 18 Kohlenstoffatomen,
- R²: einen Alkylrest mit 1 bis 12 Kohlenstoffatomen, einen Alkenyl- oder Alkinylrest mit 2 bis 12 Kohlenstoffatomen, einen Alkoxyalkylrest mit 3 bis 12 Kohlenstoffatomen, einen Cycloalkylrest mit 4 bis 12 Kohlenstoffatomen, einen Arylrest mit 6 bis 12 Kohlenstoffatomen, einen Aralkylrest mit 7 bis 12 Kohlenstoffatomen oder einen Aryloxyalkylrest mit 8 bis 12 Kohlenstoffatomen und
- n: eine ganze Zahl größer als 3
bedeuten.

Besonders bevorzugt sind solche Verbindungen der allgemeinen Formel I, in denen
- R¹: eine gegebenenfalls verzweigte Alkylen-, Cycloalkylen-, Cycloalkylendialkyl-, Alkenylen- oder Alkinylengruppe mit bis zu 8 Kohlenstoffatomen, in der gegebenenfalls ein Kohlenstoffatom durch ein Sauerstoff- oder Schwefelatom ersetzt ist oder eine Arylendialkylgruppe mit 8 bis 12 Kohlenstoffatomen,
- R²: einen Alkyl-, Alkenyl-, Alkinyl-, Cycloalkyl- oder Alkoxyalkylrest mit bis zu 6 Kohlenstoffatomen, einen Aryl-, Arylalkyl- oder Aryloxyalkylrest mit 7 bis 12 Kohlenstoffatomen,
- R³: einen Alkylrest mit 1 bis 6 Kohlenstoffatomen, einen gegebenenfalls substituierten ein- oder mehrkernigen Arylrest mit 6 bis 12 Kohlenstoffatomen und
- n: eine ganze Zahl zwischen 3 und 50
bedeuten.

Ganz besonders bevorzugt sind solche Verbindungen der allgemeinen Formel I, in denen
- R¹: eine gegebenenfalls verzweigte Alkylengruppe mit 2 bis 4 Kohlenstoffatomen,
- R²: einen Alkyl-, Alkenyl-, Alkinyl- oder Cycloalkylrest mit bis zu 6 Kohlenstoffatomen und
- R³: einen Alkylrest mit 1 bis 6 Kohlenstoffatomen oder einen gegebenenfalls substituierten sechsgliedrigen Arylrest
bedeuten.

Im einzelnen kann unter den besonders bevorzugten Verbindungen der Rest R³ z.B. die folgende Bedeutung haben:
Methyl, Ethyl, n-Propyl, Isopropyl, n-Butyl, Isobutyl, n-Hexyl, Phenyl, 4-Acetylamino-phenyl, 4-Acetoxy-phenyl, 3- und 4-Benzyloxy-phenyl, 3-Benzyloxy-4- und 4-Benzyloxy-3-methoxyphenyl, Biphenyl-4-yl, 3,5-Bis-trifluormethylphenyl, 2-, 3- und 4-Fluor-phenyl, 2-, 3-und 4-Chlorphenyl, 2-, 3- und 4-Brom-phenyl, 4-Jodphenyl, 2-, 3- und 4-Cyanophenyl, 2-, 3- und 4-Methyl-, -Ethyl-, -Propyl-, -Isopropyl-, -Butyl-, -Isobutyl-, -t-Butyl-, -Pentyl-, -Hexyl-, -Cyclohexyl-, -Nonyl- und -Dodecylphenyl, 2-, 3- und 4-Methoxy-, -Ethoxy-, -Isopropoxy-, -Butoxy-, -Pentyloxy-, -Octyloxy- und -Decyloxyphenyl, 2,3-, 2,4-, 2,6-, 3,4- und 3,5-Difluor-, -Dichlor- und -Dibromphenyl, 3-(3,4-Dichlorphenoxy)-phenyl, 3-(3,5-Dichlor-phenoxy)-phenyl, 3-Brom-4-fluor-phenyl, 5-Brom-2,4-dimethoxy-phenyl, 2-Chlor-6-fluor-phenyl, 2-Chlor-5-, 2-Chlor-6-, 4-Chlor-3- und 5-Chlor-2-nitrophenyl, 3-(4-Chlor-phenoxy)-phenyl, 3,4-Bis-benzyloxyphenyl, 2,3-, 2,4-, 2,5-, 3,4- und 3,5-Dimethoxy-, -Diethoxy-, -Dibutoxy- und Dihexyloxy-phenyl-, 2,4-Dimethoxy-3-methyl-phenyl, 2-Ethoxy-5-methoxy-phenyl, 3-chlor-4-methylphenyl, 2,4- und 2,5-Dimethylphenyl, 2-(2-Methoxy-ethyl)-, 2-(2-Methoxy-ethyl)-, und 2-(2-Butoxy-ethyl)-phenyl, 3-(2-Methoxy-ethyl)-, 3-(2-Ethoxy-ethyl)- und 3-(2-Butoxy-ethyl)-phenyl, 4-(2-Methoxyethyl)-, 4-(2-Ethoxy-ethyl)- und 4-(2-Butoxy-ethyl)phenyl, 2,6-Dinitro-phenyl, 2,4,6-Trimethyl-phenyl, 3,4,5-Trimethoxy- und -Triethoxy-phenyl, 2,3- und 3,4-Methylendioxyphenyl, 2- und 3-Thienyl, 2-Fluorenyl, 9-Anthryl, 5-Bromo-2-thienyl, 3-Methyl-2-thienyl, 5-Methyl-2-thienyl, 5-Nitrofuryl, 10-Chloro-9-anthryl und Ferrocenyl.

Diese Reste sind insbesondere deshalb bevorzugt, weil die ihnen zugrundeliegenden Ausgangsverbindungen im Handel erhältlich sind. Ihre Auswahl kann unter anderem auch nach optischen Gesichtspunkten erfolgen, d.h. es sollte darauf geachtet werden, daß die Absorption des hergestellten erfindungsgemäßen N,O-Acetals im Wellenlängenbereich der verwendeten Strahlung möglichst gering gehalten wird. Weitere Ausgangsverbindungen sind auf einfache Weise zugänglich.

Die Herstellung der erfindungsgemäßen Verbindungen gemäß der allgemeinen Formel I kann nach an sich bekannten Verfahren über eine Mehrstufensynthese erfolgen. Dazu bedarf es der Bereitstellung von geeigneten Amid-, Urethan- oder Harnstoffgruppen enthaltenden Alkoholen, deren Herstellung nachstehend beschrieben wird. Diese werden in einem Folgeschritt mit geeigneten monomeren Acetalen oder Acetalvorstufen z. B. gemäß EP-A 0 312 751 umgesetzt. Im Folgenden wird die Synthese anhand einiger erfindungsgemäßer Beispiele verdeutlicht. Gt bedeutet Gewichtsteile.

### Herstellungsbeispiel 1

1. Stufe: 88,1 Gt Ethylencarbonat wurden mit 59,1 Gt n-Propylamin versetzt. Die sich erwärmende Mischung wurde 5 Stunden bei 70 °C gerührt und im Vakuum destilliert. Farbloses Öl, das bei 102 bis 105 °C/0,06 Torr überdestilliert (N-Propyl-carbamidsäure-(2-hydroxy-ethyl)ester)
2. Stufe: 15,2 Gt Benzaldehyddimethylacetal, hergestellt durch Acetalisierung von Benzaldehyd und Methanol, wurden mit 29,4 Gt des vorstehend beschriebenen Urethanalkohols in 200 Gt Toluol in Gegenwart von 0,2 Gt p-Toluolsulfonsäure zum Rückfluß erhitzt. Von Zeit zu Zeit wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Trocknung wurde das Toluol im Rotationsverdampfer abdestilliert und der verbleibende Rückstand im Hochvakuum (0,01 Torr) bei 200 °C kondensiert. Es wurde eine oligomere Verbindung der Formel I erhalten, in der R¹ eine Ethylengruppe, X die Gruppe -O-CO-, R² eine n-Propylgruppe und R₃ eine Phenylgruppe bedeuten (Verbindung 1).

### Herstellungsbeispiel 2

Verbindung 1 wurde auch auf folgendem Weg erhalten:
15,2 Gt Benzaldehyddimethylacetal wurden mit 14,7 Gt des vorstehend beschriebenen Urethanalkohols vermischt und nach Zusatz von 0,2 Gt p-Toluolsulfonsäure langsam auf 160 °C erwärmt. Das entstehende Methanol wurde abdestilliert. Nach etwa 2 Stunden wurde ein Vakuum (0,01 Torr) angelegt und auf 200 °C erhitzt. Das Gemisch wurde diesen Bedingungen weitere 2 Stunden ausgesetzt. Es verblieb ein hochviskoses Öl, welches in seinen Eigenschaften mit denen der Verbindung 1 praktisch identisch war.

### Herstellungsbeispiel 3

Verbindung 1 konnte auch auf folgendem Weg erhalten werden:
15,2 Gt Benzaldehyd-dimethylacetal, hergestellt durch Acetalisierung von Benzaldehyd mit Methanol, wurden mit 29,4 Gt des in Beispiel 1 beschriebenen Urethanalkohols in 200 Gt Toluol in Gegenwart von 0,2 Gt p-Toluolsulfonsäure zum Rückfluß erhitzt. Von Zeit zu Zeit wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Einengen des Lösemittels verblieb ein öliger Rückstand, der aus heißem Diisopropylether umgelöst wurde. Beim Abkühlen kristallisierten weiße Kristalle mit einem Schmelzpunkt von 54,5 °C aus der Lösung aus. 19,1 Gt dieser Kristalle wurden unter Rühren mit 7,6 Gt Benzaldehyddimethylacetal und 0,2 Gt p-Toluolsulfonsäure langsam auf 160 °C erwärmt. Das entstehende Methanol wurde abdestilliert. Nach etwa 2 Stunden wurde ein Vakuum (0,01 Torr) angelegt und auf 200 °C erhitzt. Das Gemisch wurde diesen Bedingungen weitere 2 Stunden ausgesetzt. Es verblieb ein hochviskoses Öl, welches in seinen Eigenschaften mit denen der Verbindung 1 praktisch identisch war.

### Herstellungsbeispiel 4

1. Stufe: 88,1 Gt Ethylencarbonat wurden mit 101,2 Gt n-Hexylamin versetzt. Die sich erwärmende Mischung wurde 5 Stunden bei 70 °C gerührt und im Vakuum destilliert. Farbloses Öl, das bei 117 bis 119 °C/0,05 Torr überdestilliert (N-Hexyl-carbamidsäure-(2-hydroxy-ethyl)ester)
2. Stufe: 19,6 Gt Piperonal-dimethylacetal wurden mit 38,0 Gt des vorstehend beschriebenen Urethanalkohols in 200 Gt Toluol in Gegenwart von 0,2 Gt p-Toluolsulfonsäure zum Rückfluß erhitzt. Von Zeit zu Zeit wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Trocknung wurde das Toluol im Rotationsverdampfer abdestilliert und der verbleibende Rückstand im Hochvakuum (0,01 Torr) bei 200 °C kondensiert. Es wurde eine oligomere Verbindung der Formel I erhalten, in der R¹ eine Ethylengruppe, X die Gruppe -O-CO-, R² die n-Hexylgruppe und R³ die 4-Piperonylgruppe bedeuten (Verbindung 2).

### Herstellunosbeispiel 5

1. Stufe: 88,06 Gt Ethylencarbonat wurden mit 73,1 Gt i-Butylamin versetzt. Die sich erwärmende Mischung wurde 5 Stunden bei 70 °C gerührt und im Vakuum destilliert. Farbloses Öl, das bei 101 bis 104 °C/0,06 Torr überdestilliert (N-Isobutyl-carbamidsäure-(2-hydroxy-ethyl)ester)
2. Stufe: 18,6 Gt 4-Chlor-benzaldehyddimethylacetal wurden mit 32,2 Gt des vorstehend beschriebenen Urethanalkohols in 200 Gt Toluol in Gegenwart von 0,2 Gt p-Toluolsulfonsäure zum Rückfluß erhitzt. In Abständen wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Trocknung wurde das Toluol abgedampft und der verbleibende Rückstand im Hochvakuum (0,01 Torr) bei 200 °C kondensiert. Es wurde eine oligomere Verbindung der Formel I erhalten, in der R¹ eine Ethylengruppe, X die Gruppe -O-CO-, R² eine i-Butylgruppe und R³ eine 4-Chlor-phenyl-gruppe bedeuten (Verbindung 3).

### Herstellungsbeispiel 6

1. Stufe: 20,6 Gt Aminopropanol wurden in 250 Gt Diethylether und 25 Gt Isopropanol bei Raumtemperatur tropfenweise mit 21,3 Gt Propylisocyanat in 25 Gt Diethylether versetzt. Das ausgefallene Produkt wurde abgesaugt. (N-(3-Hydroxy-propyl)-N'-propyl-harnstoff, weißes Pulver, Schmelzpunkt 76 °C)
2. Stufe: 15,2 Gt Benzaldehyd-dimethylacetal wurden mit 32,2 Gt des vorstehend beschriebenen Harnstoffalkohols in 200 Gt Toluol in Gegenwart von 0,2 Gt p-Toluolsulfonsäure zum Rückfluß erhitzt. Von Zeit zu Zeit wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und bis zum Neutralpunkt mit Wasser gewaschen. Nach Trocknung wurde das Toluol abgedampft und der verbleibende Rückstand im Hochvakuum (0,01 Torr) bei 220 °C kondensiert. Es wurde eine oligomere Verbindung der Formel I erhalten, in der R¹ eine Propylengruppe, X die Gruppe -NH-CO-, R² eine n-Propylgruppe und R³ eine Phenylgruppe bedeuten (Verbindung 4).

### Herstellungsbeispiel 7

1. Stufe: Ein Gemisch aus 200 Gt Ethylenglykol und 0,5 Gt Diazabicyclo[2,2,2]octan in 200 Gt Tetrahydrofuran wurde tropfenweise mit 139,1 Gt Butylisocyanat versetzt. Die sich erwärmende Mischung wurde 5 Stunden bei 70 °C gerührt und im Vakuum destilliert. Farbloses Öl, das bei 110 bis 112 °C/0,03 Torr überdestilliert (N-Butyl-carbamidsäure-(2-hydroxy-ethyl)-ester)
2. Stufe: 19,6 Gt Piperonaldimethylacetal wurden mit 32,2 Gt des vorstehend beschriebenen Urethanalkohols in 200 Gt Toluol in Gegenwart von 0,2 Gt p-Toluolsulfonsäure zum Rückfluß erhitzt. In Abständen wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Trocknung wurde das Toluol abdestilliert und der verbleibende Rückstand im Hochvakuum (0,01 Torr) bei 200 °C kondensiert. Es wurde eine oligomere Verbindung der Formel I erhalten, in der R¹ eine Ethylengruppe, X die Gruppe -O-CO-, R² eine n-Butylgruppe und R³ eine [1,3]Benzodioxol-5-yl-gruppe bedeuten (Verbindung 5).

### Herstellungsbeispiel 8

1. Stufe: 88,06 Gt Ethylencarbonat wurden mit 73,1 Gt Isobutylamin versetzt. Die sich erwärmende Mischung wurde 5 Stunden bei 70 °C gerührt und im Vakuum destilliert. Farbloses öl, das bei 101 bis 104 °C/0,06 Torr überdestilliert.
2. Stufe: 25,8 Gt 4-Benzyloxy-benzaldehyd-dimethylacetal wurden mit 32,2 Gt des vorstehend beschriebenen Urethanalkohols in 200 Gt Toluol in Gegenwart von 0,2 Gt p-Toluolsulfonsäure zum Rückfluß erhitzt. In Abständen wurde das entstandene Toluol/Methanol-Gemisch abdestilliert, bis nach etwa 6 Stunden die Kopftemperatur über längere Zeit konstant bei 110 °C blieb. Das Gemisch wurde abgekühlt, mit verdünnter Natronlauge und dann bis zum Neutralpunkt mit Wasser gewaschen. Nach Trocknung wurde das Toluol abgedampft und der verbleibende Rückstand im Hochvakuum (0,01 Torr) bei 200 °C kondensiert. Es wurde eine oligomere Verbindung der Formel I erhalten, in der R¹ eine Ethylengruppe, X die Gruppe -O-CO-, R² eine Butylgruppe und R³ eine 4-Benzyloxyphenylgruppe bedeuten (Verbindung 6).

Weitere Verbindungen wurden in entsprechender Weise nach bekannten Methoden hergestellt; eine Auflistung besonders bevorzugter Verbindungen zur Herstellung strahlungsempfindlicher Gemische ist in den beiliegenden Formelzeichnungen aufgeführt.

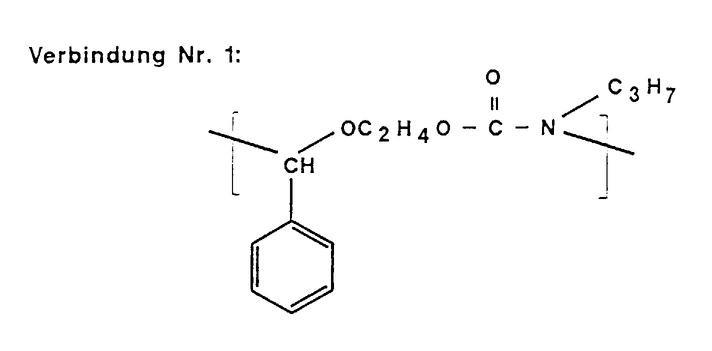

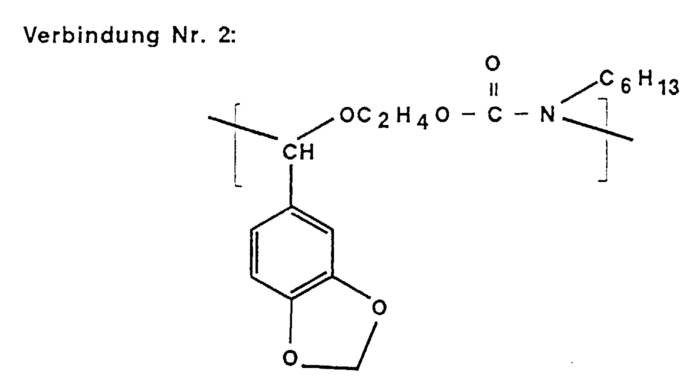

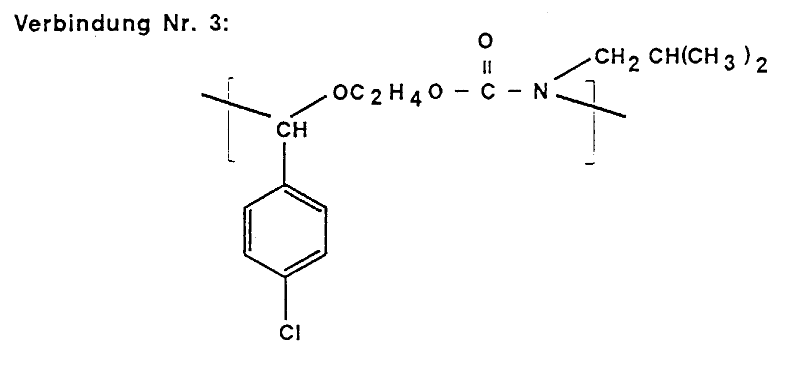

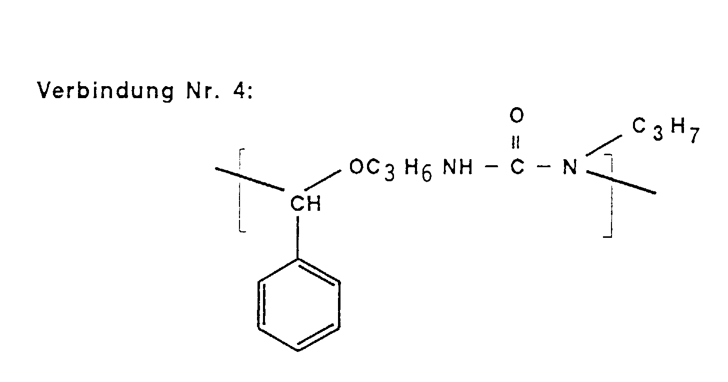

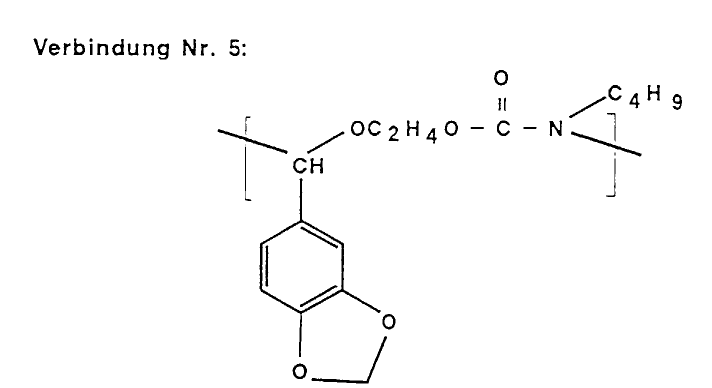

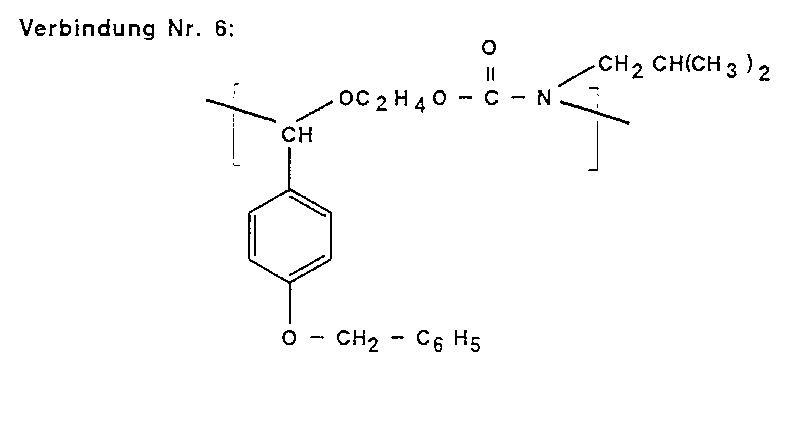

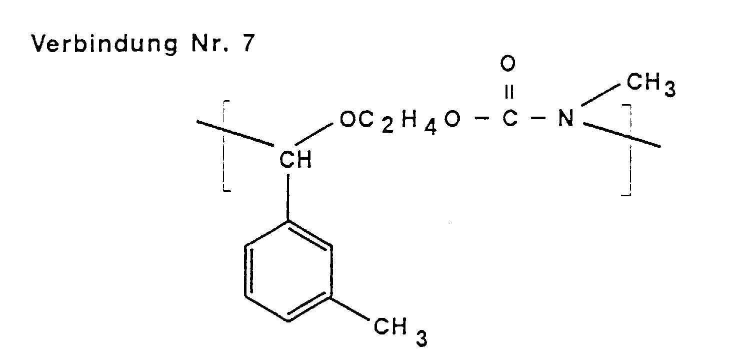

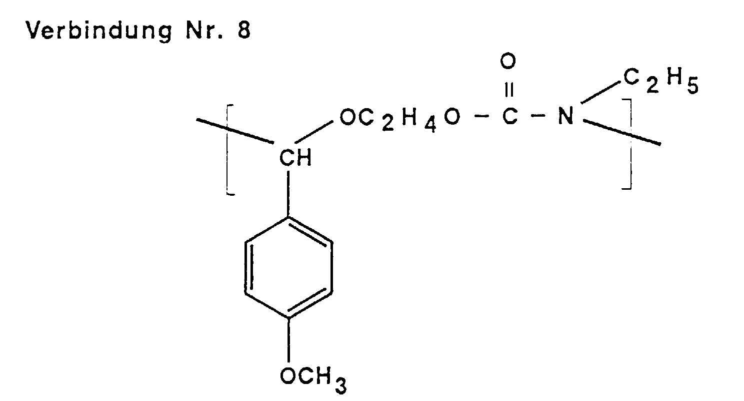

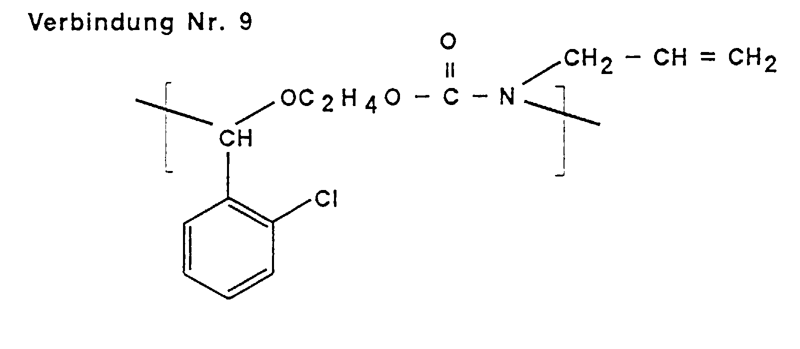

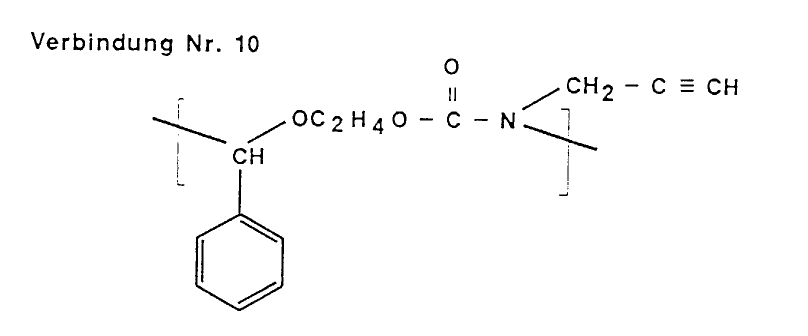

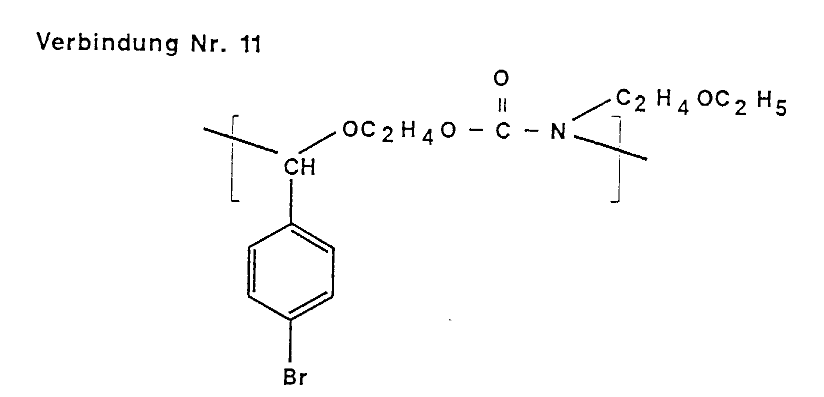

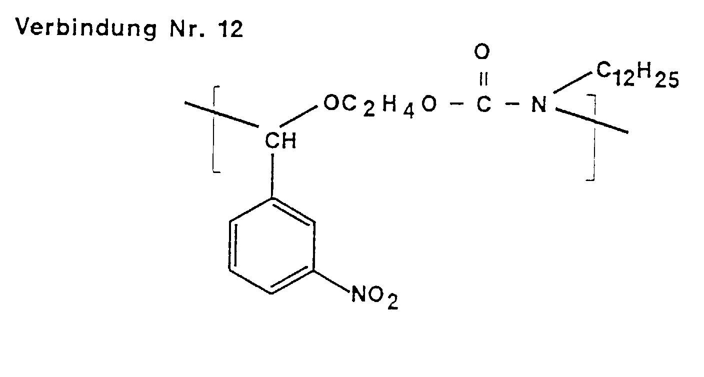

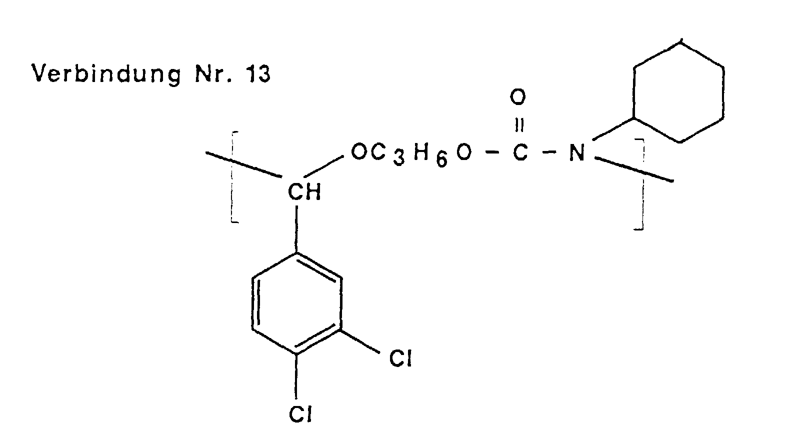

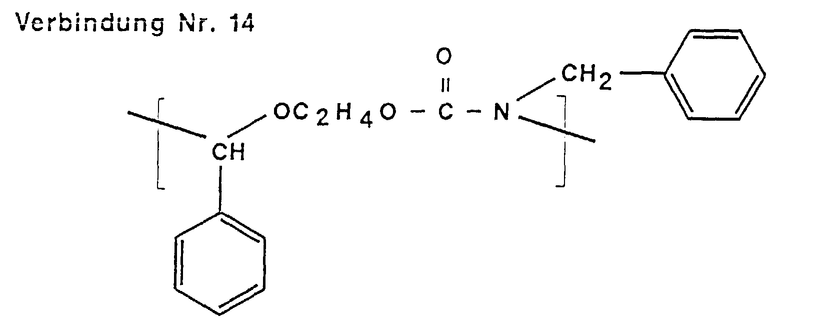

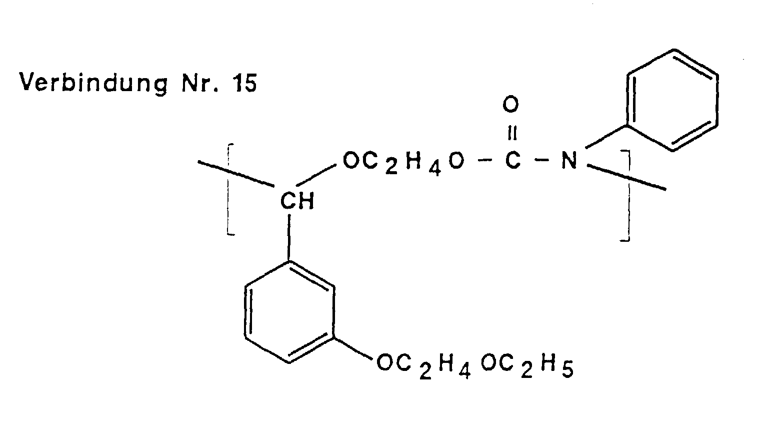

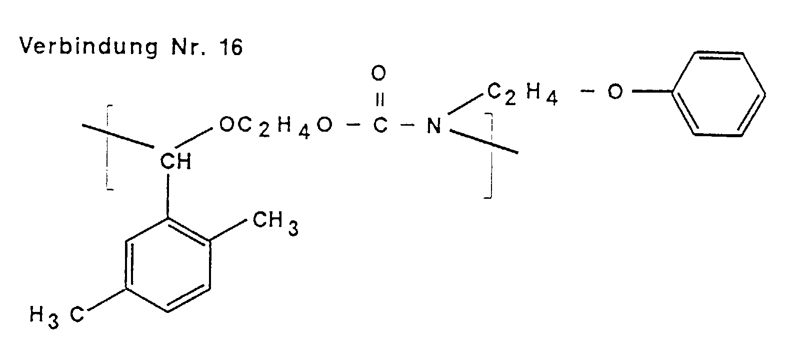

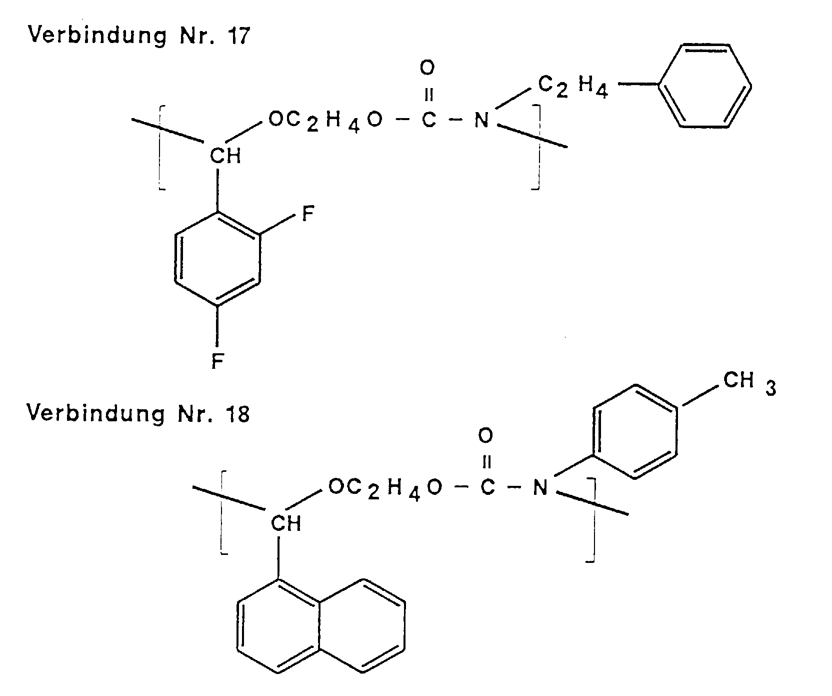

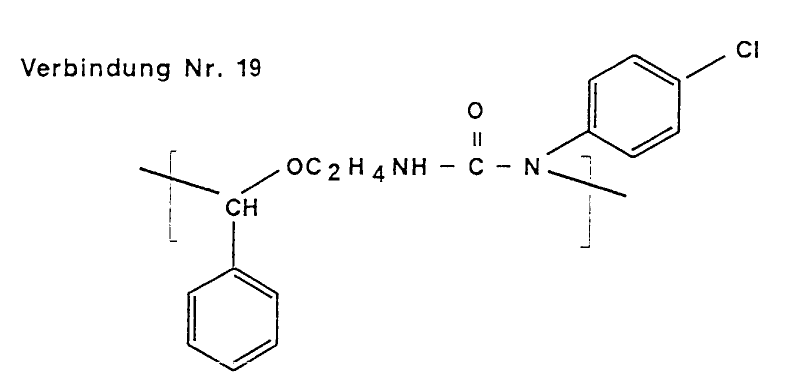

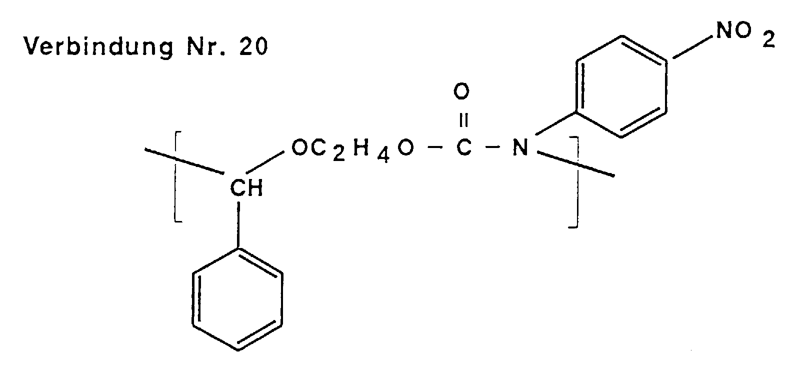

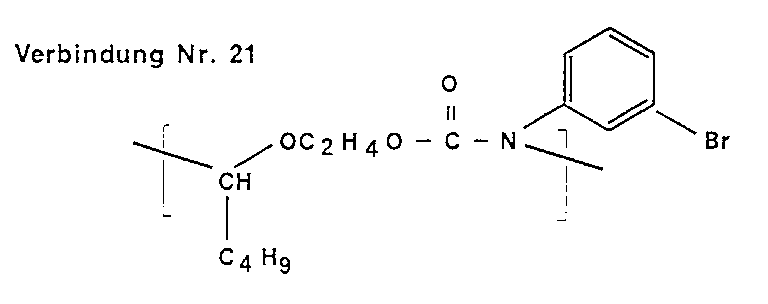

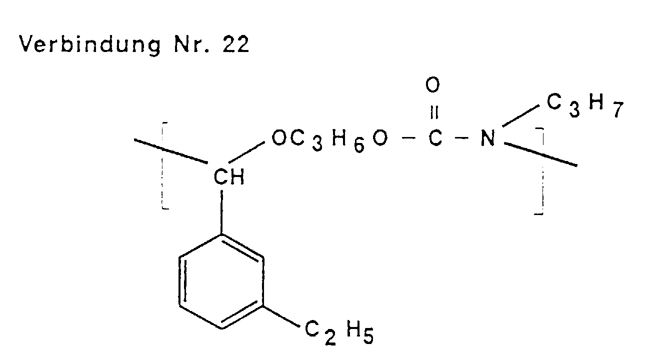

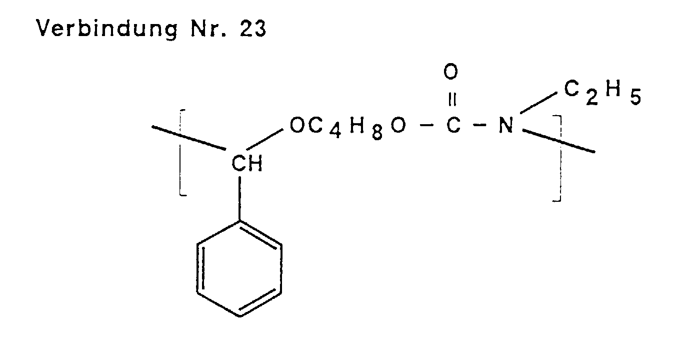

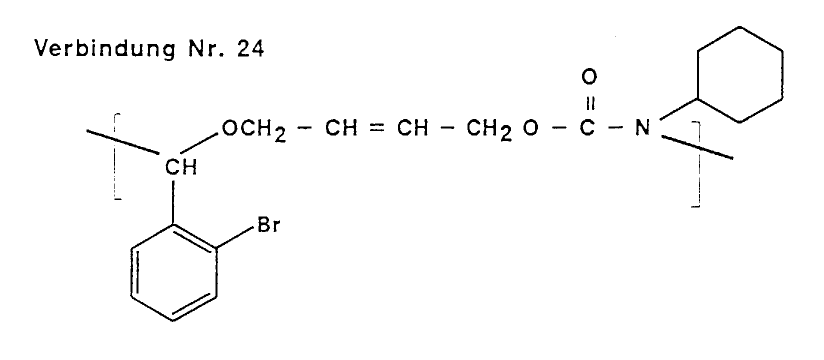

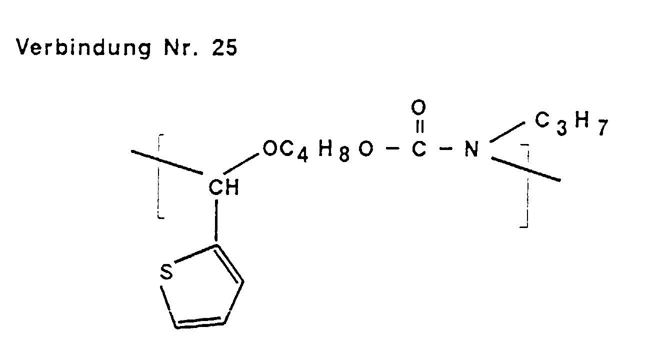

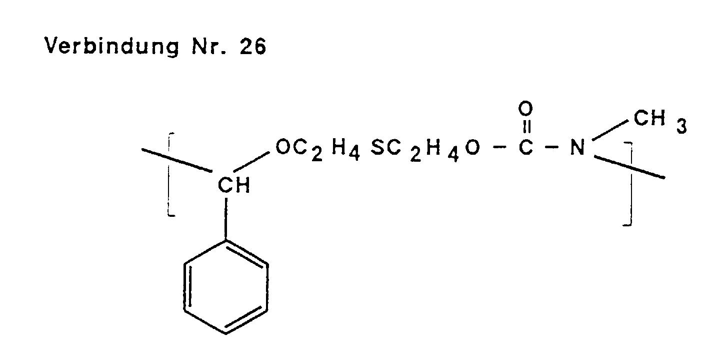

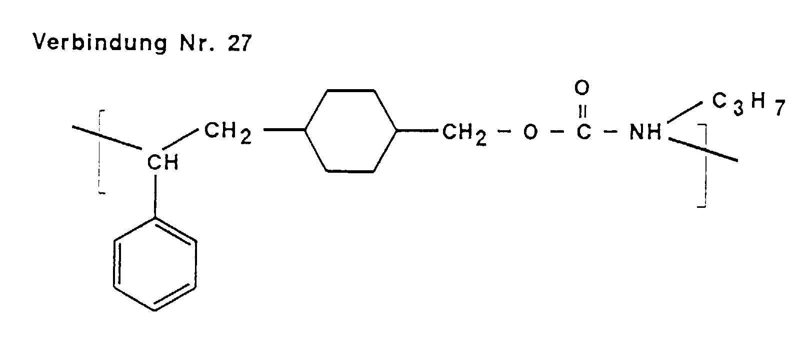

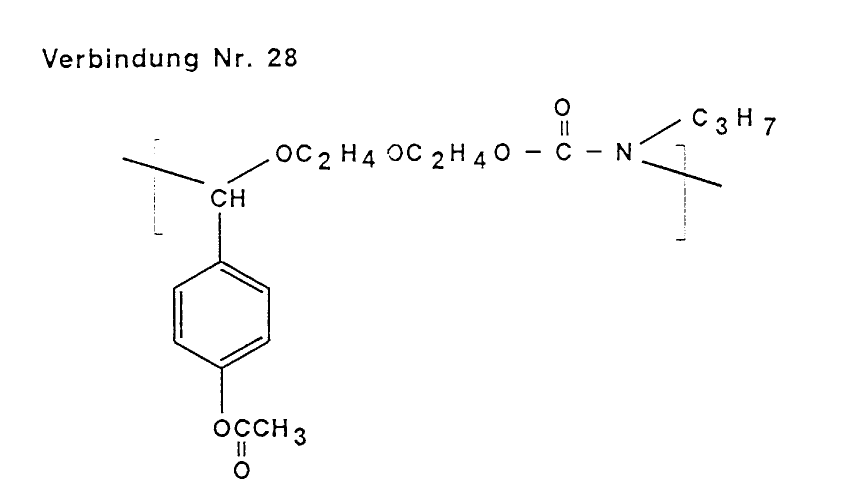

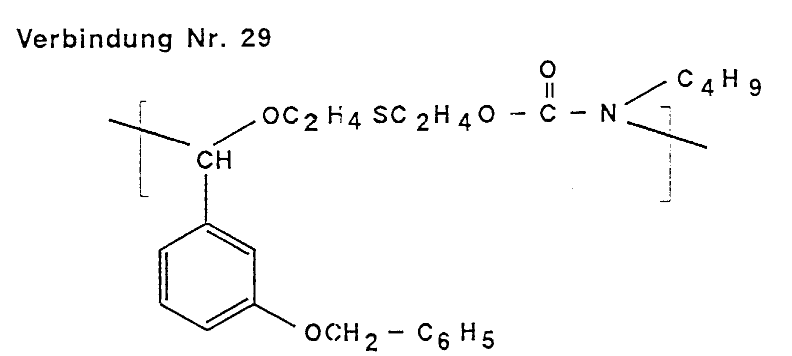

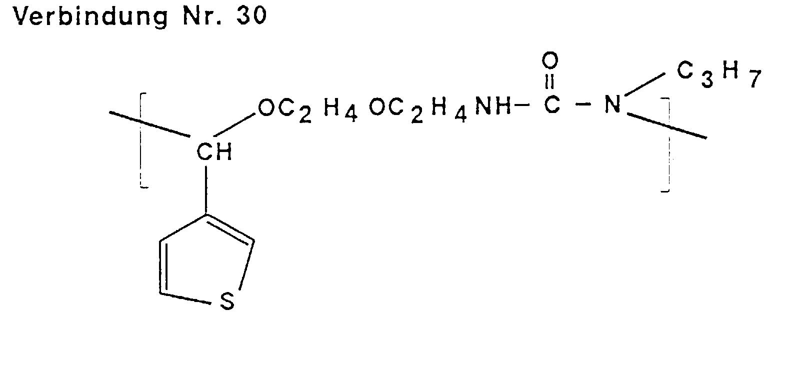

Weitere Verbindungen der allgemeinen Formel 1, die ähnlich günstige Eigenschaften aufweisen, sind in nachstehender Tabelle zusammengefaßt:

| Nr. | X | R¹ | R² | R³ |
|---|---|---|---|---|
| 31 | O-CO | C₂H₄ | C₃H₇ | C₂H₅ |
| 32 | CO | C₃H₆ | C₃H₇ | C₄H₉ |
| 33 | O-CO | C₂H₄ | C₃H₇ | C₄H₉ |
| 34 | O-CO | C₃H₆ | C₃H₇ | C₄H₉ |
| 35 | NH-CO | C₃H₆ | C₄H₉ | C₄H₉ |
| 36 | NH-CO | CH(CH₃)CH₂ | C₃H₇ | C₄H₉ |
| 37 | O-CO | C₂H₄ | C₃H₆Cl | C₄H₉ |
| 38 | NH-CO | C₃H₆ | C₃H₇ | 4-Bromphenyl |
| 39 | O-CO | C₂H₄ | C₃H₇ | 4-Methoxyphenyl |
| 40 | CO | C₃H₆ | C₃H₇ | 4-Tolyl |
| 41 | O-CO | C₄H₈ | C₄H₉ | 2-Naphthyl |
| 42 | NH-CO | C₃H₆ | C₃H₇ | 4-Nitrophenyl |
| 43 | NH-CO | C₂H₄ | C₃H₇ | 3-Methoxyphenyl |
| 44 | O-CO | C₃H₆ | C₃H₇ | 3,4-Dichlorphenyl |
| 45 | O-CO | C₂H₄ | C₃H₇ | 2,6-Dichlorphenyl |
| 46 | O-CO | CH(CH₃)CH₂ | C₃H₇ | 2,4-Difluorphenyl |
| 47 | O-CO | C₃H₆ | C₃H₇ | 2,3,5-Trichlorphenyl |
| 48 | O-CO | C₂H₄ | C₃H₇ | Ferrocenyl |

In der US-A-3,981,913 werden mit CH₂OH-Gruppen und Oxyalkylenresten substituierte Harnstoffderivate offenbart, die jedoch zur Behandlung von Textilien eingesetzt werden.

Erfindungsgemäß wird ferner ein strahlungsempfindliches Gemisch vorgeschlagen, das als wesentliche Bestandteile
(a) ein in Wasser unlösliches, in wäßrig-alkalischen Lösungen lösliches oder zumindest quellbares Bindemittel,
(b) eine Verbindung, die unter Einwirkung von aktinischer Strahlung eine starke Säure bildet, und
(c) eine Verbindung enthält, die mindestens eine durch Säure spaltbare C-O-C-Bindung aufweist und deren Säurespaltprodukte sich in wäßrig-alkalischen Lösungen leichter lösen als die Verbindung selbst.

Das erfindungsgemäße Gemisch ist dadurch gekennzeichnet, daß die durch Säure spaltbare Verbindung (c) ein oligomeres N,O-Acetal der vorstehend beschriebenen Formel I ist.

Das erfindungsgemäße strahlungsempfindliche Gemisch zeichnet sich durch eine hohe Empfindlichkeit über einen weiten Spektralbereich aus. Es zeigt eine hohe thermische Stabilität und schafft die Möglichkeit, auch feinste Strukturen einer Vorlage strukturgenau wiederzugeben. Dies beruht auf der Eigenschaft des die erfindungsgemäßen Verbindungen enthaltenden Gemischs, daß sich eine ungewöhnlich hohe Differenzierung der Löslichkeit zwischen Bild- und Nichtbildbereichen erreichen läßt.

Der Gehalt an säurespaltbarem Material (c) in dem erfindungsgemäßen strahlungsempfindlichen Gemisch sollte bei 1 bis 60 Gew.-%, vorzugsweise bei 5 bis 50 Gew.-%, jeweils bezogen auf das Gesamtgewicht des Gemisches, liegen.

Den im erfindungsgemäßen strahlungsempfindlichen Gemisch enthaltenen säurespaltbaren Verbindungen c) werden photolytische Säurespender b) zugesetzt, wozu sich Onium-Salze, wie Diazonium-, Phosphonium-, Sulfonium- und Jodonium-Salze von nicht nukleophilen Säuren, z.B. von HSbF₆, HAsF₆ oder HPF₆ [J.V. Crivello, Polym. Eng. Sci., 23, 953 (1983)], Halogenverbindungen (EP-A 0 232 972, DE-A 15 72 089, DE-A 18 17 540, DE-A 19 49 010, US-A 3 912 606, DE-A 23 17 846), insbesondere Trichlormethyltriazinderivate (US-A 3 515 552, US-A 3 536 489, US-A 3 779 778, DE-A 27 18 259, DE-A 33 37 024, DE-A 33 33 450, DE-A 23 06 248, DE-A 22 43 621, DE-A 12 98 414) oder Trichlormethyloxadiazolderivate (DE-A 30 21 590, DE-A 30 21 599, DE-A 28 51 472, DE-A 29 49 396, DE-A 33 33 450, EP-A 0 135 348), o-Chinondiazidsulfochloride oder Organometall-Organohalogen-Kombinationen eignen. Obwohl sich mit derartigen Verbindungen sehr gute Ergebnisse erzielen lassen, sind diese insgesamt nicht bevorzugt, da in solchen strahlungsempfindlichen Gemischen die Bildung von stark korrodierend wirkenden Säuren stattfindet. Es sind vielmehr solche photolytische Säurespender bevorzugt, die bei der Belichtung Sulfonsäuren bilden. Als Beispiele für solche Verbindungen seien die in den DE-A 39 30 087 und 39 30 086 beschriebenen α-Carbonyl-α-sulfonyldiazomethane oder α,α-Bissulfonyldiazo-methane, Nitrobenzylsulfonate (F.M. Houlihan et al., J. Photopolym. Sci. Techn., 3, 259, 1990; T. Yamaoka et al., J. Photopolym. Sci. Techn., 3, 275, 1990), Pyrogallolsulfonate [T. Ueno et al., Chemical Amplification Positive Resist Systems Using Novel Sulfonates as Acid Generators, in "Polymers for Microelectronics - Science and Technology", Hrsg. Y. Tabata et al., Kodansha-Weinheim-New York, 1989, S. 66-67] oder Iminosulfonate (M. Shirai et al., J. Photopolym. Sci. Techn., 3, 301, 1990) erwähnt. Besonders bevorzugt sind die in den gleichzeitig eingereichten Anmeldungen P 41 12 967.9, P 41 12 966.0 und P 41 12 965.2 erwähnten N-Sulfonyloxygruppen enthaltenden α-Pyridone.

Die photolytischen Säurespender werden dem Gemisch im allgemeinen zu 0,2 bis 25 Gew-% zugesetzt. Bevorzugt sind Anteile von 0,5 bis 15 Gew.-%, besonders bevorzugt 1 bis 10 Gew.-%. Eine genaue Festlegung dieser Mengen kann nur in einer konkreten Formulierung erfolgen und differiert von Typ zu Typ. Die photolytischen Säurespender haben Absorptionsmaxima im Bereich zwischen 200 und 500 nm. Durch sie wurde daher die spektrale Empfindlichkeit der erfindungsgemäßen Gemische festgelegt, und sie können so ausgewählt werden, daß man praxisgerechte Empfindlichkeiten im Bereich der technisch wichtigen Strahlungsquellen, z.B. g-line (436 nm), i-line (365 nm) oder UV2 (248 nm) erzielen kann. Die erfindungsgemäß verwendbaren säurespaltbaren Verbindungen (c) können dabei so ausgewählt werden, daß sie in diesen Bereichen praktisch keine Eigenabsorption aufweisen. Durch dem Fachmann bekannte Eingriffe, z. B. durch spektrale Sensibilisierung läßt sich der Empfindlichkeitsbereich der säurespendenden Verbindungen (b) weiter vergrößern, so daß auch Strahlungsquellen im sichtbaren oder im kurzwelligen Bereich der Röntgenstrahlung Verwendung finden können. Schließlich lassen sich auch andere Strahlungsquellen, wie Elektronen- oder Ionenstrahlen zur bildmäßigen Differenzierung des erfindungsgemäßen Gemischs einsetzen, insbesondere dann, wenn man hochaktive Säurespender, wie sie beispielsweise in der EP-A 0 318 649 beschrieben werden, einsetzt.

Den erfindungsgemäßen Gemischen können gegebenenfalls andere säurespaltbare Verbindungen zugefügt werden; dabei haben sich vor allem folgende Verbindungsklassen bewährt:
(1) solche mit mindestens einer Orthocarbonsäureester- und bzw. oder Carbonsäureamidacetalgruppierung, wobei die Verbindungen auch polymeren Charakter haben und die genannten Gruppierungen als verknüpfende Elemente in der Hauptkette oder als seitenständige Substituenten auftreten können (DE-A 26 10 842 und 29 28 636),
(2) Oligomer- oder Polymerverbindungen mit wiederkehrenden Acetal- und/oder Ketalgruppierungen in der Hauptkette (DE-A 23 06 248 und 27 18 254),
(3) Verbindungen mit mindestens einer Enolether- oder N-Acyliminocarbonatgruppierung (EP-A 0 006 626 und 0 006 627),
(4) cyclische Acetale oder Ketale von β-Ketoestern oder -amiden (EP-A 0 202 196),
(5) Verbindungen mit Silylethergruppierungen (DE-A 35 44 165 und 36 01 264),
(6) Verbindungen mit Silyl-enolethergruppierungen (DE-A 37 30 785 und 37 30 783),
(7) Monoacetale bzw. Monoketale, deren Aldehyd- bzw. Ketonkomponente eine Löslichkeit im Entwickler zwischen 0,1 und 100 g/l aufweisen (DE-A 37 30 787),
(8) Ether auf der Basis tertiärer Alkohole (US-A 4 603 101) und
(9) Carbonsäureester und Carbonate tertiärer, allylischer oder benzylischer Alkohole [US-A 4 491 628 und J. M. Fréchet et al., J. Imaging Sci. 30, 59-64 (1986)].

Es können auch Mischungen der genannten säurespaltbaren Verbindungen eingesetzt werden. Unter diesen bevorzugt verwendbar sind allerdings säurespaltbare Verbindungen, die einem der obengenannten Typen (1) bis (9) zuzuordnen sind, und darunter besonders diejenigen, die eine durch Säure spaltbare C-O-C-Bindung aufweisen. Unter diesen besonders bevorzugt sind diejenigen Verbindungen, die den Typen (1), (2), (7) und (9) angehören. Unter Typ (2) sind besonders die polymeren Acetale hervorzuheben; von den säurespaltbaren Verbindungen des Typs (7) insbesondere diejenigen, deren Aldehyd- bzw. Ketonkomponente einen Siedepunkt oberhalb 150 °C, vorzugsweise oberhalb 200 °C, aufweist. Insgesamt jedoch sind solche Mischungen nicht bevorzugt.

Die Verbindung (c) bzw. die Kombination von Verbindungen (c) ist in einer Konzentration von 1 bis 60 Gew.-% enthalten.

Das erfindungsgemäße strahlungsempfindliche Gemisch enthält ferner mindestens ein polymeres, in Wasser unlösliches, in wäßrig-alkalischen Lösungen dagegen lösliches, zumindest quellbares Bindemittel. Das Bindemittel zeichnet sich im besonderen dadurch aus, daß es mit den übrigen Bestandteilen des erfindungsgemäßen strahlungsempfindlichen Gemisches gut verträglich ist und insbesondere im Wellenlängenbereich von 190 bis 550 nm eine möglichst geringe Eigenabsorption, d.h. eine hohe Transparenz, aufweist. Bindemittel auf der Basis von Novolak-Kondensationsharzen, die in der Regel in Kombination mit Naphthochinondiaziden als photoaktive Komponenten eingesetzt werden, erfüllen diese Bedingung nicht. Zwar lassen Novolak-Kondensationsharze nach bildmäßiger Belichtung in den belichteten Bereichen eine Erhöhung der Löslichkeit gegenüber wäßrig-alkalischen Entwicklern erkennen, doch ist ihre Eigenabsorption im Bereich der für die Bestrahlung gewünschten kurzen Wellenlänge unerwünscht hoch.

Für die klassischen Anwendungen, d. h. mit Lichtquellen im nahen UV-Bereich, sind insbesondere Bindemittel auf der Basis von Novolak-Kondensationsharzen geeignet, die in der Regel in Kombination mit Naphthochinondiaziden als photoaktive Komponenten eingesetzt worden sind. Solche Phenol-Formaldehyd-Kondensate sind mehrfach beschrieben worden und können als phenolische Komponente Phenol, die drei stellungsisomeren Kresole oder andere Alkylphenole, z. B. Xylenole, als Komponenten enthalten. Neben Formaldehyd können auch andere Aldehyde zur Herstellung des Polymeren herangezogen werden. Allerdings lassen sich auch die nachstehend beschriebenen, Hydroxylgruppen enthaltenden Polymeren für Bestrahlungen mit nahem UV-Licht verwenden, wobei diese den oben bezeichneten Novolak-Polymeren in einem Anteil von bis zu 50%, bevorzugt bis zu 20%, zugemischt sein können.

Für die Anwendungen im UV2-Bereich können Novolak-Kondensationsharze aber in Mischung mit anderen, als Bindemittel geeigneten Harzen mit höherer Transparenz eingesetzt werden. Die Mischungsverhältnisse richten sich dabei vorwiegend nach der Art des mit dem Novolakharz zu mischenden Bindemittels. Insbesondere spielen dessen Grad an Eigenabsorption im genannten Wellenlängenbereich, aber auch die Mischbarkeit mit den anderen Bestandteilen des strahlungsempfindlichen Gemisches eine entscheidende Rolle. Im allgemeinen kann aber das Bindemittel des erfindungsgemäßen strahlungsempfindlichen Gemisches bis zu 50 Gew.-%, insbesondere bis zu 20 Gew.-%, eines Novolak-Kondensationsharzes enthalten.

Als Bindemittel geeignet sind Homo- oder Copolymere des p-Hydroxystyrols sowie seiner Alkylderivate, z. B. des 3-Methyl-4-hydroxystyrols, des 3,5-Dimethyl-4-hydroxystyrols oder des 2,3-Dimethyl-4-hydroxystyrols sowie Homo- oder Copolymere anderer Vinylphenole, z. B. des 2- oder 3-Hydroxystyrols, oder des 4-Methyl-3-hydroxystyrols oder die Ester der (Meth)acrylsäure mit Phenolen, z. B. Brenzkatechin, Resorcin, Hydrochinon, Pyrogallol oder Aminophenole sowie die entsprechenden Amide mit aromatischen Aminen. Als Comonomere können polymerisierbare Verbindungen wie Styrol, Methylmethacrylat, Methylacrylat oder ähnliche eingesetzt werden.

Gemische mit erhöhter Plasmabeständigkeit werden dann erhalten, wenn zur Herstellung von Copolymeren des obigen Typs Silicium enthaltende Vinylmonomere, z. B. Vinyltrimethylsilan oder Allyltrimethylsilan, verwendet werden. Die Transparenz dieser Bindemittel ist im interessierenden Bereich im allgemeinen höher, so daß eine verbesserte Strukturierung möglich ist.

Mit gleichem Erfolg lassen sich auch Homo- oder Copolymere des Maleinimids verwenden. Auch diese Bindemittel zeigen hohe Transparenz im beschriebenen Wellenlängenbereich. Als Comonomere werden auch hier bevorzugt Styrol, substituierte Styrole, Vinylphenole, Propenylphenole, Vinylether, Vinylester, Vinylsilylverbindungen oder (Meth)acrylsäureester eingesetzt.

Schließlich sind darüber hinaus auch Copolymere des Styrols mit Comonomeren verwendbar, die in wäßrig alkalischen Lösungen eine Löslichkeitserhöhung bewirken. Hierunter zählen beispielsweise Maleinsäureanhydrid, Maleinsäurehalbester oder dergleichen.

Die genannten Bindemittel können vorteilhaft auch untereinander gemischt werden, sofern sich dadurch die filmbildenden Eigenschaften und die optische Qualität des strahlungsempfindlichen Gemisches nicht verschlechtern.

Die Menge des Bindemittels beträgt im allgemeinen 40 bis 95 Gew.-%, insbesondere 40 bis 90 Gew.-%, vorzugsweise 50 bis 85Gew.-%, bezogen auf das Gesamtgewicht der strahlungsempfindlichen Mischung.

Die Extinktion des Bindemittels bzw. der Kombination von Bindemitteln (a) im Wellenlängenbereich der Empfindlichkeit der Verbindung (b) sollte weniger als 0,5 µm⁻¹ betragen.

Ferner können den erfindungsgemäßen strahlungsempfindlichen Gemischen gegebenenfalls Farbstoffe, Pigmente, Weichmacher, Netzmittel und Verlaufmittel, aber auch Polyglykole, Celluloseether, z. B. Ethylcellulose, zur Verbesserung spezieller Erfordernisse, wie Flexibilität, Haftung und Glanz, zugesetzt werden.

Soll ein Substrat beschichtet werden, so wird das erfindungsgemäße strahlungsempfindliche Gemisch zweckmäßig in einem Lösemittel oder in einer Kombination von Lösemitteln gelöst. Hierfür besonders geeignet sind Ethylenglykol, Ethylenglykolmonomethylether, Ethylenglykoldimethylether, Ethylenglykolmonoethylether oder Propylenglykolmonoalkylether, insbesondere Propylenglykolmethylether, aliphatische Ester (z. B. Ethylacetat, Hydroxyethylacetat, Alkoxyethylacetat, n-Butylacetat, Propylenglykolalkyletheracetat, insbesondere Propylenglykolmethyletheracetat oder Amylacetat), Ether (z. B. Dioxan), Ketone (z. B. Methylethylketon, Methylisobutylketon, Cyclopentanon und Cyclohexanon), Dimethylformamid, Dimethylacetamid, Hexamethylphosphorsäureamid, N-Methyl-pyrrolidon, Butyrolacton, Tetrahydrofuran und Mischungen davon. Besonders bevorzugt werden Glykolether, aliphatische Ester sowie Ketone.

Letztendlich hängt die Wahl der Lösemittel von dem angewandten Beschichtungsverfahren, der gewünschten Schichtstärke und den Trocknungsbedingungen ab. Ebenso müssen die Lösemittel chemisch neutral sein, d. h. sie dürfen nicht mit den übrigen Schichtkomponenten irreversibel reagieren.

Die mit den genannten Lösemitteln hergestellte Lösung hat in der Regel einen Feststoffgehalt von 5 bis 60 Gew.-%, vorzugsweise bis 50 Gew.-%.

Gegenstand der Erfindung ist schließlich auch ein strahlungsempfindliches Aufzeichnungsmaterial, das im wesentlichen aus einem Substrat und einer darauf befindlichen strahlungsempfindlichen Schicht aus dem erfindungsgemäßen strahlungsempfindlichen Gemisch besteht.

Als Substrate kommen alle Materialien in Frage, aus denen Kondensatoren, Halbleiter, mehrlagige gedruckte Schaltungen oder integrierte Schaltkreise bestehen bzw. hergestellt werden können. Speziell sind Siliciumsubstrate zu nennen, die auch thermisch oxidiert und/oder mit Aluminium beschichtet, aber auch dotiert sein können. Daneben sind alle anderen in der Halbleitertechnologie üblichen Substrate möglich, wie Siliciumnitrid, Galliumarsenid und Indiumphosphid. Weiterhin kommen die aus der Flüssigkristalldisplay-Herstellung bekannten Substrate in Frage, wie z. B. Glas und Indium-Zinnoxid, ferner Metallplatten und -folien-beispielsweise aus Aluminium, Kupfer, Zink-, Bimetall- und Trimetallfolien, aber auch elektrisch nichtleitende Folien, die mit Metallen bedampft sind, und Papier. Diese Substrate können thermisch vorbehandelt, oberflächlich aufgerauht, angeätzt oder, zur Verbesserung erwünschter Eigenschaften, z. B. zur Erhöhung der Hydrophilie, mit Chemikalien vorbehandelt sein.

Um der strahlungsempfindlichen Schicht einen besseren Zusammenhalt und/oder eine bessere Haftung auf der Substratoberfläche zu verleihen, kann in ihr ein Haftvermittler enthalten sein. Bei Silicium- bzw. Siliciumdioxid-Substraten kommen hierfür Haftvermittler vom Aminosilan-Typ, wie z. B. 3-Aminopropyltriethoxysilan oder Hexamethyldisilazan, in Frage.

Geeignete Träger für die Herstellung von photomechanischen Aufzeichnungsschichten, wie Druckformen für den Hochdruck, Flachdruck, Siebdruck und Flexodruck sind besonders Aluminiumplatten, die gegebenenfalls vorher anodisch oxidiert, gekörnt und/oder silikatisiert werden, daneben Zink- und Stahlplatten, die gegebenenfalls verchromt werden, sowie Kunststoffolien und Papier.

Das erfindungsgemäße Aufzeichnungsmaterial wird mit aktinischer Strahlung bildmäßig belichtet. Als aktinische Strahlung soll im Rahmen dieser Beschreibung jede Strah-lung verstanden werden, deren Energie mindestens der des kurzwelligen sichtbaren Lichts entspricht. Als Strahlungsquellen eignen sich besonders Metallhalogenidlampen, Kohlebogenlampen, Xenonlampen und Quecksilberdampflampen. Ebenso kann eine Belichtung mit energiereicher Strahlung wie Laser-, Elektronen- oder Röntgenstrahlung erfolgen. Besonders bevorzugt sind jedoch Lampen, die Licht einer Wellenlänge von 190 bis 260 nm ausstrahlen können, d.h. insbesondere Xenon- und Quecksilberdampflampen. Darüber hinaus lassen sich auch Laserlichtquellen verwenden, z. B. Excimerlaser, insbesondere KrF- oder ArF-Laser, die bei 248 bzw. 193 nm emittieren. Die Strahlungsquellen müssen in den genannten Wellenlängenbereichen eine ausreichende Emission aufweisen.

Die Stärke der lichtempfindlichen Schicht hängt vom Verwendungszweck ab. Sie beträgt im allgemeinen zwischen 0,1 und 100 µm, bevorzugt zwischen 1 und 10 µm.

Gegenstand der Erfindung ist ferner ein Verfahren zur Herstellung eines strahlungsempfindlichen Aufzeichnungsmaterials. Das Auftragen des strahlungsempfindlichen Gemisches auf das Substrat kann durch Aufsprühen, Fließbeschichten, Walzen, Schleuder- und Tauchbeschichten erfolgen. Danach wird das Lösemittel durch Verdampfen entfernt, so daß auf der Oberfläche des Substrats die strahlungsempfindliche Schicht zurückbleibt. Die Entfernung des Lösemittels kann durch Erhitzen der Schicht auf Temperaturen bis zu 150 °C gefördert werden. Das Gemisch kann aber auch zunächst auf obengenannte Weise auf einen Zwischenträger aufgetragen werden, von dem aus es unter Druck und erhöhter Temperatur auf das endgültige Trägermaterial übertragen wird. Als Zwischenträger können grundsätzlich alle auch als Trägermaterialien geeigneten Materialien Anwendung finden. Anschließend wird die Schicht bildmäßig bestrahlt. Danach behandelt man die Schicht mit einer Entwicklerlösung, die die bestrahlten Bereiche der Schicht löst und entfernt, so daß ein Abbild der bei der bildmäßigen Bestrahlung verwendeten Vorlage auf der Substratoberfläche verbleibt.

Als Entwickler eignen sich besonders wäßrige Lösungen, die Silikate, Metasilikate, Hydroxide, Hydrogen- und Dihydrogenphosphate, Carbonate oder Hydrogencarbonate von Alkalimetall-, Erdalkalimetall- und/oder Ammoniumionen enthalten, aber auch Ammoniak und dergleichen. Metallionenfreie Entwickler werden in der US-A 4 729 941, der EP-A 0 062 733, der US-A 4 628 023, der US-A 4 141 733, der EP-A 0 097 282 und der EP-A 0 023 758 beschrieben. Der Gehalt dieser Substanzen in der Entwicklerlösung beträgt im allgemeinen 0,1 bis 15 Gew.-%, vorzugweise 0,5 bis 5 Gew.-%, bezogen auf das Gewicht der Entwicklerlösung. Metallionenfreie Entwickler werden bevorzugt verwendet. Den Entwicklern können gegebenenfalls geringe Mengen eines Netzmittels zugesetzt sein, um die Ablösung der löslichen Bereiche der Schicht zu erleichtern.

Die entwickelten Schichtstrukturen können nachgehärtet werden. Dies geschieht im allgemeinen durch Erhitzen auf einer hot-plate bis zu einer Temperatur unterhalb der Fließtemperatur und anschließendes ganzflächiges Belichten mit dem UV-Licht einer Xenon-Quecksilber-Dampflampe (Bereich von 200 bis 250 nm). Durch die Nachhärtung werden die Bildstrukturen vernetzt, so daß sie im allgemeinen eine Fließbeständigkeit bis zu Temperaturen von über 200 °C aufweisen. Die Nachhärtung kann auch ohne Temperaturerhöhung allein durch Bestrahlen mit energiereichem UV-Licht erfolgen.

Verwendung findet das erfindungsgemäße strahlungsempfindliche Gemisch bei der Herstellung von integrierten Schaltungen oder von diskreten elektronischen Bausteinen mit lithographischen Prozessen. Die entwickelte Resistschicht dient dabei als Maske für die folgenden Prozeßschritte. Solche Schritte sind z. B. das Ätzen des Schichtträgers, das Implantieren von Ionen in den Schichtträger oder das Abscheiden von Metallen oder anderen Materialien auf dem Schichtträger.

Die folgenden Beispiele 1 bis 17 belegen die Eignung des erfindungsgemäßen Gemisches für Aufzeichnungsmaterialien in der Mikrolithographie unter Verwendung von Strahlung unterschiedlichster Energie. Anhand der Vergleichsbeispiele 18 bis 21 wird die Überlegenheit der erfindungsgemäßen Gemische gegenüber dem Stand der Technik belegt. Die Beispiele 22 bis 24 dokumentieren die Anwendbarkeit des Gemisches in gedruckten Schaltungen und Flachdruckplatten.

In den Beispielen sind die Mengen in der Regel als Gewichtsteile (Gt) angegeben. Wenn nichts anderes angegeben ist, sind Prozentzahlen und Mengenverhältnisse in Gewichtseinheiten zu verstehen.

### Beispiel 1

Es wurde eine Beschichtungslösung hergestellt aus

| | |
|---|---|
| 7,5 Gt | eines Kresol-Formaldehyd-Novolaks mit einem Erweichungsbereich von 105 bis 120 °C, |
| 2,0 Gt | der Verbindung 1 und |
| 0,6 Gt | α,α-Bis-(4-tert.-butyl-benzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen je Minute aufgeschleudert. Nach 1 Minute Trocknen bei 100 °C auf der hot plate wurde eine Schichtdicke von 1,1 µm erhalten.

Das Aufzeichnungsmaterial wurde bildmäßig unter einer Vorlage mit der Strahlung einer Xenon-Quecksilber-dampflampe bei 365 nm einer Energie von 80 mJ/cm² belichtet. Um die Spaltung des Lösungsinhibitors zu vervollständigen, wurde das Material 1 Minute auf 60 °C erwärmt.

Entwickelt wurde das Aufzeichnungsmaterial mit einem Entwickler folgender Zusammensetzung:

| | |
|---|---|
| 5,3 Gt | Natriummetasilikat x 9 H₂O, |
| 3,4 Gt | Trinatriumphosphat x 12 H₂O, |
| 0,3 Gt | Natriumdihydrogenphosphat und |
| 191 Gt | vollentsalztes Wasser. |

Nach einer Entwicklungsdauer von 60 s erhielt man ein fehlerfreies Abbild der Maske mit steilen Resistflanken, wobei auch Strukturen < 0.55 µm detailgetreu aufgelöst waren. Eine Untersuchung der Flanken der Resistprofile mittels Rasterelektronenmikroskopie belegte, daß diese praktisch senkrecht zur Substratoberfläche ausgerichtet waren. Der Kontrast betrug 3,8.

Der Kontrast eines Positivresists, γₚ ist definiert als

γₚ = 1 / (log Dₚ - log Dₚ^{o}) = [log(Dₚ/Dₚ^{o})]⁻¹

wobei Dₚ^{o} die Einstrahlungsdosis ist, bei welcher der Entwickler beginnt, den belichteten Film anzugreifen, und Dₚ den Resistaufpunkt (= Resistempfindlichkeit) darstellen. Eine genaue Beschreibung dieser Größe ist im Aufsatz von L.F. Thompson und M.J. Bowden "Resist Processing" (Introduction to Microlithography, Theory, Materials and Processing, Herausgeber C.G. Willson, L.F. Thompson und M.J. Bowden, ACS Symp. Ser., 219, 164 ff (1983), American Chemical Society, Washington) gegeben worden.

### Beispiel 2

Es wurde eine Beschichtungslösung hergestellt aus

| | |
|---|---|
| 7,5 Gt | eines Copolymeren aus Styrol/p-Hydroxystyrol (Molverhältnis 20:80) mit einem mittleren Molekulargewicht von 32.000, |
| 2,0 Gt | der Verbindung 2 und |
| 0,5 Gt | α,α-Bis-(4-tert.-butyl-benzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.000 Umdrehungen aufgeschleudert. Nach 1 Minute Trocknen bei 100 °C auf der hot plate wurde eine Schichtdicke von 1,12 µm erhalten.

Das Aufzeichnungsmaterial wurde bildmäßig unter einer Vorlage mit Strahlung einer Xenon-Quecksilberdampflampe bei 260 nm mit einer Energie von 92 mJ/cm² belichtet und anschließend 1 Minute auf 65 °C erwärmt. Dann wurde es mit einem wäßrigen Entwickler verarbeitet, der 2,38% Tetramethylammoniumhydroxid enthielt. Nach 60 s erhielt man ein fehlerfreies Abbild der Maske mit hoher Flankenstabilität. Es wurde ein Kontrastwert von 5,6 bestimmt, wobei auch hier Strukturen < 0,55 µm detailgetreu aufgelöst waren.

### Beispiel 3

Es wurde eine Beschichtungslösung hergestellt aus

| | |
|---|---|
| 7,5 Gt | eines Poly(brenzkatechin-monomethacrylats) mit einem Erweichungsbereich von 125 bis 135 °C, |
| 2,0 Gt | der Verbindung 3 und |
| 0,4 Gt | 2-(4-Ethoxy-naphthalin-1-yl)-4,6-bis-trichloromethyl-1,3,5-triazin in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen aufgeschleudert. Nach 1 Minute Trocknen bei 100 °C auf der hot plate wurde eine Schichtdicke von 1,1 µm erhalten.

Ein derartig beschichteter Wafer wurde unter einer Vorlage mit Licht einer Wellenlänge von 405 nm und einer Energie von 100 mJ/cm² bestrahlt. Anschließend wurde das Material 1 Minute auf 60 °C erwärmt. Nach der Entwicklung wurde ein originalgetreues Abbild der Vorlage erhalten.

### Beispiel 4:

Der Versuch von Beispiel 3 wurde wiederholt, jedoch wurde Licht einer Wellenlänge von 436 nm verwendet. Um ein kantenscharfes Abbild der Vorlage zu erhalten, mußte eine Belichtungsenergie von 135 mJ/cm² eingesetzt werden.

### Beispiel 5

Es wurde eine Beschichtungslösung hergestellt aus

| | |
|---|---|
| 7,5 Gt | eines Copolymeren aus Styrol und Maleimid (Molverhältnis 1:1) mit einem Erweichungsbereich von 165 bis 180 °C, |
| 2,0 Gt | der Verbindung 3 und |
| 0,6 Gt | Benzolsulfonyl-(4-chlor-benzoyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.700 Umdrehungen aufgeschleudert. Nach 1 Minute Trocknen bei 100 °C wurde eine Schichtdicke von 0,98 µm erhalten.

Das Aufzeichnungsmaterial wurde bildmäßig unter einer Vorlage mit der Strahlung einer Xenon-Quecksilberdampflampe bei 260 nm mit einer Energie von 120 mJ/cm² belichtet. Dann wurde das Material 2 Minuten auf 65 °C erwärmt.

Entwickelt wurde das Aufzeichnungsmaterial mit einer 0,02 n wäßrigen Lösung von Tetramethylammoniumhydroxid, wobei die belichteten Bereiche innerhalb von 60 s rückstandsfrei abgelöst wurden. Es wurde ein fehlerfreies Abbild der Maske mit steilen Resistflanken erhalten. Der Dunkelabtrag war < 20 nm; auch Strukturen < 0,6 µm waren detailgetreu aufgelöst.

### Beispiel 6

Es wurde eine Beschichtungslösung hergestellt aus

| | |
|---|---|
| 7,5 Gt | des Copolymeren aus Beispiel 5, |
| 2,0 Gt | der Verbindung 4 und |
| 0,4 Gt | 4-(2-[2]Furyl-vinyl)-6-trichloromethyl-2-pyron in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100 °C wurde eine Schichtdicke von 1,00 µm erhalten.

Das Aufzeichnungsmaterial wurde bildmäßig unter einer Vorlage mit Strahlung einer Xenon-Quecksilberdampflampe bei 260 nm mit einer Energie von 112 mJ/cm² belichtet und anschließend 1 Minute auf 60 °C erwärmt.

Entwickelt wurde das Aufzeichnungsmaterial mit einer 0,02 n wäßrigen Lösung von Tetramethylammoniumhydroxid, wobei die belichteten Bereiche innerhalb von 60 s rückstandsfrei abgelöst wurden und ein detailgetreues Abbild der Vorlage erhalten wurde. Der gemessene Kontrast betrug ca. 8; die Kantensteilheit des Bildes war dementsprechend ausgezeichnet.

### Beispiel 7

Es wurde eine Beschichtungslösung hergestellt aus

| | |
|---|---|
| 7,5 Gt | des in Beispiel 2 beschriebenen Copolymeren, |
| 2,0 Gt | der Verbindung 11 und |
| 0,3 Gt | Triphenylsulfonium-trifluoromethansulfonat in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen bei 100 °C wurde eine Schichtdicke von 1,04 µm erhalten.

Das Aufzeichnungsmaterial wurde bildmäßig unter einer Vorlage mit Strahlung einer Xenon-Quecksilberdampflampe bei 260 nm mit einer Energie von 112 mJ/cm² belichtet. Anschließend wurde es 1 Minute auf 60 °C erwärmt.

Entwickelt wurde das Aufzeichnungsmaterial mit einer 0,27 n wäßrigen Lösung von Tetramethylammoniumhydroxid, wobei die belichteten Bereiche innerhalb von 60 s rückstandsfrei abgelöst wurden und ein detailgetreues Abbild der Vorlage erhalten wurde. Linien und Spalten bis herab zu 0,5 µm wurden maskengetreu wiedergegeben.

### Beispiel 8

Das Aufzeichnungsmaterial aus Beispiel 7 wurde mit Synchrotronstrahlung (BESSY, Berlin, 754 MeV) durch eine Gold-auf-Silizium-Maske mit einer Dosis von 120 mJ/cm² bestrahlt. Den experimentellen Aufbau findet man bei A. Heuberger, Microelectr. Eng., 3, 535 (1985). Das belichtete Material wurde 30 Minuten bei Raumtemperatur liegen gelassen. Nach Entwicklung mit dem in Beispiel 7 beschriebenen Entwickler und einer Entwicklungsdauer von 120 s erhielt man ein fehlerfreies Bild der Maske bis herab zu Strukturen < 0,4 µm. Der Kontrast wurde zu > 8 bestimmt; die Resistflanken waren praktisch senkrecht zur planaren Substratoberfläche.

### Beispiel 9

Es wurde eine Beschichtungslösung hergestellt aus

| | |
|---|---|
| 7,5 Gt | eines Formaldehyd/m-Kresol/p-Kresol-Novolaks mit einem Erweichungspunkt von 100 bis 110 °C, |
| 2,0 Gt | der Verbindung 14 und |
| 0,3 Gt | 2-(4-Methoxystyryl)-4,6-bis-trichloromethyl-1,3,5-triazin in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen aufgeschleudert. Nach 1 Minute Trocknen bei 100 °C wurde eine Schichtdicke von 1,05 µm erhalten.

Das Aufzeichnungsmaterial wurde bildmäßig unter einer Vorlage mit Strahlung einer Wellenlänge von 365 nm mit einer Energie von 95 mJ/cm² belichtet und anschließend 1 Minute bei 65 °C erwärmt.

Entwickelt wurde das Aufzeichnungsmaterial mit einem Entwickler nachstehender Zusammensetzung:

| | |
|---|---|
| 5,5 Gt | Natriummetasilikat x 9 H₂O, |
| 3,4 Gt | Trinatriumphosphat x 12 H₂O und |
| 0,4 Gt | Mononatriumphosphat, wasserfrei, in |
| 90,7 Gt | vollentsalztem Wasser, |

wobei die belichteten Bereiche innerhalb von 60 s rückstandsfrei abgelöst wurden und ein detailgetreues Abbild der Vorlage erhalten wurde. Linien und Spalten bis herab zu 0,5 µm wurden maskengetreu wiedergegeben.

### Beispiel 10

Es wurde eine Beschichtungslösung hergestellt aus

| | |
|---|---|
| 6,0 Gt | eines Homopolymerisats aus 3-Methyl-4-hydroxystyrol mit einem Erweichungspunkt von 155 bis 160 °C und einem Molekulargewicht von 22.000, |
| 3,5 Gt | der Verbindung 1 und |
| 0,1 Gt | 1,2,3-Tris-methansulfonyloxy-benzol in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.000 Umdrehungen aufgeschleudert. Nach 1 Minute Trocknen bei 100 °C wurde eine Schichtdicke von 1,05 µm erhalten.

Das Aufzeichnungsmaterial wurde bildmäßig unter einer Vorlage mit Strahlung einer Wellenlänge von 248 nm mit einer Energie von 25 mJ/cm² belichtet. Anschließend wurde das Aufzeichnungsmaterial 1 Minute auf 60 °C erwärmt.

Entwickelt wurde das Aufzeichnungsmaterial mit einem wäßrigen Entwickler, der 2.38% Tetramethylammoniumhydroxid enthielt. Bereits nach wenigen Sekunden konnte das vorlagengetreue Abbild erhalten werden, in dem auch Details < 0,5 µm aufgelöst waren. Der Kontrast wurde zu 4,5 bestimmt.

### Beispiel 10a

Wie in Beispiel 10 beschrieben, wurden 5 Wafer beschichtet, jedoch wurden anstelle der dort angegebenen Menge der Verbindung 1 0,3, 0,6, 0,9, 1,2 und 1,5 Gt eingesetz. Die Proben wurden wie in Beispiel 10 beschrieben verarbeitet. Die Ergebnisse sind in den Tabellen nach Vergleichsbeispiel 21 angegeben.

### Beispiel 11

Das in Beispiel 10 beschriebene Gemisch wurde wie dort aufgetragen, belichtet und nacherwärmt. Die Entwicklung erfolgte mit dem dort beschriebenen Entwickler, dem 10 % Isopropanol zugesetzt worden waren. Es wurden Details bis herab zu 0,3 µm dem Vorbild getreu abgebildet. Die Profile auch der kleinsten Strukturen waren praktisch vertikal.

### Beispiel 12

Es wurde eine Beschichtungslösung hergestellt aus

| | |
|---|---|
| 6,0 Gt | des in Beispiel 10 angegebenen Homopolymerisats, |
| 3,5 Gt | der Verbindung 1 und |
| 0,2 Gt | 4-Methyl-6-(4-methoxystyryl)-N-methansulfonyloxy-α-pyridon in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.100 Umdrehungen aufgeschleudert. Nach 1 Minute Trocknen bei 100 °C wurde eine Schichtdicke von 1,05 µm erhalten.

Das Aufzeichnungsmaterial wurde bildmäßig unter einer Vorlage mit der Strahlung eines KrF-Excimer-Lasers bei 248 nm mit einer Energie von 65 mJ/cm² belichtet. Anschließend wurde das Aufzeichnungsmaterial 1 Minute auf 60 °C erwärmt.

Entwickelt wurde das Aufzeichnungsmaterial mit einem wäßrigen Entwickler, der 2,38 % Tetramethylammoniumhydroxid enthielt. Bereits nach wenigen Sekunden konnte das vorlagengetreue Abbild erhalten werden, in dem auch Details von weniger als 0,5 µm aufgelöst waren.

### Beispiel 13

Das in Beispiel 12 beschriebene Material wurde mit Licht einer Wellenlänge von 240 bis 260 nm bestrahlt und wie dort beschrieben weiterverarbeitet. Um ein getreues Abbild der Vorlage bis herab zu 0,6 µm zu erhalten war eine Dosis von 45 mJ/cm² notwendig.

### Beispiel 14

Das in Beispiel 12 beschriebene Material wurde mit Licht einer Wellenlänge von 365 nm bestrahlt und wie dort beschrieben weiterverarbeitet. Um ein getreues Abbild der Vorlage bis herab zu 0,6 µm zu erhalten war eine Dosis von 85 mJ/cm² notwendig.

### Beispiel 15

Das in Beispiel 12 beschriebene Material wurde mit Licht einer Wellenlänge von 405 nm bestrahlt und wie dort beschrieben weiterverarbeitet. Um ein getreues Abbild der Vorlage bis herab zu 0,65 µm zu erhalten war eine Dosis von 97 mJ/cm² notwendig.

### Beispiel 16

Das in Beispiel 12 beschriebene Material wurde mit Licht einer Wellenlänge von 436 nm bestrahlt und wie dort beschrieben weiterverarbeitet. Um ein getreues Abbild der Vorlage bis herab zu 0,65 µm zu erhalten war eine Dosis von 122 mJ/cm² notwendig.

### Beispiel 17 und Vergleichsbeispiele 18 bis 21

Es wurden je fünf Beschichtungslösungen der nachstehend bezeichneten fünf Grundrezepturen hergestellt, wobei der Lösungsinhibitor in einer Menge von 5 Gew.-%, 10 Gew.-%, 15 Gew.-%, 20 Gew.-% und 25 Gew.-%, bezogen auf die Menge des verwendeten Polymers eingesetzt wurde (= 0,3 Gt, 0,6 Gt, 0,9 Gt, 1,2 Gt, 1,5 Gt):

### Beispiel 17

| | |
|---|---|
| 6,0 Gt | des in Beispiel 10 angegebenen Homopolymerisats, |
| x Gt | der Verbindung 1 und |
| 0,2 Gt | 2-(4-Methoxy-styryl)-4,6-bis-trichlormethyl-1,3,5-triazin in |
| 42 Gt | Propylenglykol-monomethylether-acetat. |

### Vergleichsbeispiel 18

| | |
|---|---|
| 6,0 Gt | des in Beispiel 10 angegebenen Homopolymerisats, |
| x Gt | eines monomomeren Acetals hergestellt aus 1 mol Benzaldehyd und 2 mol Phenoxyethanol und |
| 0,2 Gt | 2-(4-Methoxy-styryl)-4,6-bis-trichlormethyl-1,3,5-triazin in |
| 42 Gt | Propylenglykol-monomethylether-acetat. |

### Vergleichsbeispiel 19

| | |
|---|---|
| 6,0 Gt | des in Beispiel 10 angegebenen Homopolymerisats, |
| x Gt | eines oligomeren Acetals hergestellt aus 1 mol Benzaldehyd und 1 mol Diethylenglykol und |
| 0,2 Gt | 2-(4-Methoxy-styryl)-4,6-bis-trichlormethyl-1,3,5-triazin in |
| 42 Gt | Propylenglykol-monomethylether-acetat. |

### Vergleichsbeispiel 20

| | |
|---|---|
| 6,0 Gt | des in Beispiel 10 angegebenen Homopolymerisats, |
| x Gt | eines oligomeren Acetals aus 1 mol Benzaldehyd und 1 mol Butin-1,4-diol und |
| 0,2 Gt | 2-(4-Methoxy-styryl)-4,6-bis-trichlormethyl-1,3,5-triazin in |
| 42 Gt | Propylenglykol-monomethylether-acetat. |

### Vergleichsbeispiel 21

| | |
|---|---|
| 6,0 Gt | des in Beispiel 10 angegebenen Homopolymerisats, |
| x Gt | eines monomomeren Acetals hergestellt aus 1 mol Benzaldehyd mit 2 mol 1,4-Bis-hydroxymethyl-cyclohexan und |
| 0,2 Gt | 2-(4-Methoxy-styryl)-4,6-bis-trichlormethyl-1,3,5-triazin in |
| 42 Gt | Propylenglykol-monomethylether-acetat. |

Die Lösungen wurden durch ein Filter mit einem Porendurchmesser von 0,2 µm filtriert und jeweils auf zwei mit einem Haftvermittler (Hexamethyldisilazan) behandelte Wafer aufgeschleudert, wobei die Bedingungen so gewählt wurden, daß nach dem Trocknen bei 100 °C eine Schichtdicke von 1,00 ± 0,05 µm erhalten wurde.

Dann wurden die Wafer in einen 0,27 mol/l Tetramethylammoniumhydroxid enthaltenden wäßrigen Entwickler 1 Minute bei 21° C eingetaucht und der Schichtabtrag mit Hilfe eines Schichtdickenmeßgeräts bestimmt. Das reine Polymer hatte in diesem Entwickler eine Abtragsrate von 327 nm/min. Es wurden folgende Werte für die Dunkelabtragsraten der verschiedenen Mischungen erhalten (nm/min):

| Beispiel Nr. | Konzentration an Lösungsinhibitor | | | | |
|---|---|---|---|---|---|
| | 5% | 10% | 15% | 20% | 25% |
| 10 | 103 | 39 | 6 | 8 | 6 |
| 17 | 92 | 45 | 10 | 10 | 8 |
| 18 | 370 | 225 | 157 | 143 | 182 |
| 19 | 340 | 309 | 299 | 266 | 287 |
| 20 | 192 | 152 | 96 | 107 | 163 |
| 21 | 197 | 145 | 114 | 97 | 65 |

Aus diesem Ergebnis ist ersichtlich, daß die erfindungsgemäßen oligomeren N,O-Acetale eine deutlich verbesserte Inhibierungswirkung für Polyhydroxystyrol-Polymere zeigen. Ähnliche Ergebnisse konnten auch mit anderen phenolischen Polymeren erhalten werden.

Anschließend wurden die verbliebenen Wafer durch einem KrF-Excimer-Laser mit einer Dosis von 100 mJ/cm² bestrahlt, 1 min auf 60° C erwärmt und der Hellabtrag bestimmt (nm/min):

| Beispiel Nr. | Konzentration an Lösungsinhibitor | | | | |
|---|---|---|---|---|---|
| | 5% | 10% | 15% | 20% | 25% |
| 10 | 450 | 600 | 780 | 900 | 977 |
| 17 | 412 | 450 | 617 | 822 | 897 |
| 18 | 470 | 521 | 640 | 842 | 912 |
| 19 | 320 | 350 | 345 | 375 | 360 |
| 20 | 400 | 510 | 590 | 690 | 820 |
| 21 | 420 | 478 | 534 | 590 | 700 |

Daraus ergibt sich, daß die Gemische, die die erfindungsgemäßen N,O-Acetale enthalten, nach Belichtung sehr gut lösungspromovierend wirken. Das Verhältnis der Abtragsraten zwischen belichteten und unbelichteten Schichtbereichen ist in ihrem Fall von allen Beispielen mit Abstand am größten, d.h. sie erlauben eine sehr gute Differenzierung zwischen Bild- und Nichtbildbereichen.

### Beispiel 22

Es wurde eine Beschichtungslösung hergestellt aus

| | |
|---|---|
| 7,5 Gt | eines Copolymeren aus 3-Methyl-4-hydroxystyrol/4-Hydroxystyrol (Molverhältnis 2:1) mit einem mittleren Molekulargewicht von 18.000, |
| 2,0 Gt | der Verbindung 1 (Molekulargewicht ca. 3000), |
| 0,2 Gt | α,α-Bis-(4-methylbenzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.200 Umdrehungen aufgeschleudert. Nach 1 Minute Trocknen auf der hot plate wurde eine Schichtdicke von 1,03 µm erhalten.

Das Aufzeichnungsmaterial wurde bildmäßig unter einer Vorlage mit der Strahlung eines KrF-Excimer-Lasers unter Verwendung eines Belichtungsgerätes PAS 5000/70 (Firma ASM-Lithography) mit einer Energie von 34 mJ/cm² belichtet und anschließend 1 Minute auf 60 °C erwärmt.

Entwickelt wurde das Aufzeichnungsmaterial mit einem wäßrigen Entwickler, der 2,38 % Tetramethylammoniumhydroxid enthielt. Nach 60 s erhielt man ein fehlerfreies Abbild der Maske, wobei auch Strukturen bis herab zu 0,35 µm detailgetreu aufgelöst waren.

### Beispiel 23

Es wurde eine Beschichtungslösung hergestellt aus

| | |
|---|---|
| 7,5 Gt | eines Copolymeren aus 3-Methyl-4-hydroxystyrol/4-Hydroxystyrol (Molverhältnis 1:1) mit einem mittleren Molekulargewicht von 17.000, |
| 2,0 Gt | der Verbindung 1 (Molekulargewicht ca. 5000), |
| 0,2 Gt | α,α-Bis-(4-chlorbenzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen auf der hot plate wurde eine Schichtdicke von 0,98 µm erhalten.

Das Aufzeichnungsmaterial wurde bildmäßig unter einer Vorlage mit der Strahlung eines KrF-Excimer-Lasers unter Verwendung eines Belichtungsgerätes PAS 5000/70 (Firma ASM-Lithography) mit einer Energie von 38 mJ/cm² belichtet und anschließend 1 Minute auf 60 °C erwärmt.

Entwickelt wurde das Aufzeichnungsmaterial mit einem wäßrigen Entwickler, der 2,38 % Tetramethylammoniumhydroxid enthielt. Nach 60 s erhielt man ein fehlerfreies Abbild der Maske, wobei auch Strukturen bis herab zu 0,30 µm detailgetreu aufgelöst waren. Der Flankenwinkel der Strukturen lag im Bereich von 90° ± 3°.

### Beispiel 24

Es wurde eine Beschichtungslösung hergestellt aus

| | |
|---|---|
| 4,5 Gt | eines Homopolymeren aus 3-Methyl-4-hydroxystyrol mit einem mittleren Molekulargewicht von 22.000, |
| 3,0 Gt | eines Homopolymeren aus 4-Hydroxystyrol mit einem mittleren Molekulargewicht von 30.000 (PHS der Hoechst Celanese Corporation), |
| 2,0 Gt | der Verbindung 1 (Molekulargewicht ca. 5000), |
| 0,3 Gt | α,α-Bis-(4-brombenzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen auf der hot plate wurde eine Schichtdicke von 0,94 µm erhalten.

Das Aufzeichnungsmaterial wurde bildmäßig unter einer Vorlage mit der Strahlung eines KrF-Excimer-Lasers unter Verwendung eines Belichtungsgerätes PAS 5000/70 (Firma ASM-Lithography) mit einer Energie von 30 mJ/cm² belichtet und anschließend 1 Minute auf 60 °C erwärmt.

Entwickelt wurde das Aufzeichnungsmaterial mit einem wäßrigen Entwickler, der 2,38 % Tetramethylammoniumhydroxid enthielt. Während die Nichtbildbereiche einen Abtrag von < 20 nm/min aufwiesen, war der Entwicklerabtrag in den belichteten Bereichen > 10.000 nm/min. Nach 60 s erhielt man wiederum ein fehlerfreies Abbild der Maske, wobei auch Strukturen bis herab zu 0,30 µm detailgetreu aufgelöst waren. Der Flankenwinkel der Strukturen lag im Bereich von 90° ± 3°.

### Beispiel 25

Es wurde eine Beschichtungslösung hergestellt aus

| | |
|---|---|
| 3,0 Gt | eines Homopolymeren aus 3-Methyl-4-hydroxystyrol mit einem mittleren Molekulargewicht von 22.000, |
| 4,5 Gt | eines Homopolymeren aus 4-Hydroxystyrol mit einem mittleren Molekulargewicht von 30.000 (PHS der Hoechst Celanese Corporation), |
| 2,0 Gt | der Verbindung 1 (Molekulargewicht ca. 5.000), |
| 0,3 Gt | α,α-Bis-(4-chlorbenzolsulfonyl)-diazomethan in |
| 42 Gt | Propylenglykol-monomethylether-acetat. |

Die Lösung wurde durch ein Filter mit einem Porendurchmesser von 0,2 µm filtriert und auf einen mit einem Haftvermittler (Hexamethyldisilazan) behandelten Wafer bei 3.500 Umdrehungen aufgeschleudert. Nach 1 Minute Trocknen auf der hot plate wurde eine Schichtdicke von 0,90 µm erhalten.

Das Aufzeichnungsmaterial wurde bildmäßig unter einer Vorlage mit der Strahlung eines KrF-Excimer-Lasers unter Verwendung eines Belichtungsgerätes PAS 5000/70 (Firma ASM-Lithography) mit einer Energie von 30 mJ/cm² belichtet und anschließend 1 Minute auf 60 °C erwärmt.

Entwickelt wurde das Aufzeichnungsmaterial mit einem wäßrigen Entwickler, der 2,38 % Tetramethylammoniumhydroxid enthielt. Während die Nichtbildbereiche einen Abtrag von < 20 nm/min aufwiesen, war der Entwicklerabtrag in den belichteten Bereichen > 10.000 nm/min.

Nach 60 s erhielt man ein fehlerfreies Abbild der Maske, wobei auch Strukturen bis herab zu 0,35 µm detailgetreu aufgelöst waren. Der Flankenwinkel der Strukturen lag im Bereich von 90° ± 3°.

### Beispiel 26

Der beschichtete Wafer des Beispiels 23 wurde mit dem dort beschriebenen Belichter durch eine phasenverschiebende Maske belichtet. Unter den in diesem Beispiel beschriebenen Bedingungen ließen sich Strukturen bis herab zu 0,16 µm maßstabgetreu und mit praktisch vertikalen Resistprofilen abbilden.

### Beispiel 27

Der beschichtete Wafer des Beispiels 24 wurde mit dem dort beschriebenen Belichter durch eine phasenverschiebende Maske belichtet. Unter den in diesem Beispiel beschriebenen Bedingungen ließen sich Strukturen bis herab zu 0,16 µm maßstabgetreu und mit praktisch vertikalen Resistprofilen abbilden.

### Beispiel 28

Für die Herstellung einer Offsetdruckplatte wurde eine Aluminiumfolie mit mechanisch aufgerauhter und anodisierter Oberfläche mit einer Beschichtungslösung nachstehender Zusammensetzung schleuderbeschichtet:

| | |
|---|---|
| 7,5 Gt | des in Beispiel 1 angegebenen Novolaks, |
| 2,3 Gt | der Verbindung 1, |
| 0,3 Gt | 2-(4-Methoxy-styryl)-4,6-bis-trichlormethyl-1,3,5-triazin und |
| 0,05 Gt | Kristallviolettbase in |
| 90 Gt | Propylenglykol-monomethylether-acetat. |

Nach dem Trocknen der Schicht (Trockengewicht ca. 2,5 g/m²) wurde unter einer positiven Testvorlage 30 s belichtet, 15 min bei Raumtemperatur gelagert und mit einem Entwickler folgender Zusammensetzung entwickelt:

| | |
|---|---|
| 0,5 Gt | Natriumhydroxid, |
| 0,8 Gt | Natriummetasilikat x 9 H₂O und |
| 1,0 Gt | 2-n-Butoxy-ethanol in |
| 97,7 Gt | vollentsalztem Wasser. |

Nach Abspülen mit Wasser wurde die Platte durch Überwischen mit 1%iger Phosphorsäure druckfertig gemacht. Nach dem Einspannen in eine Druckmaschine wurden 55.000 einwandfreie Abdrucke der Vorlage erhalten.

### Beispiel 29

Die Lösung eines Positiv-Trockenresists wurde durch Bereitung nachstehender Zusammensetzung hergestellt:

| | |
|---|---|
| 12,5 Gt | des in Beispiel 9 beschriebenen Novolaks, |
| 10,0 Gt | der Verbindung 7, |
| 0,5 Gt | 4-(2-[2]Furyl-vinyl)-6-trichlormethyl-2-pyron und |
| 0,1 Gt | Kristallviolett in |
| 25 Gt | Butanon. |

Eine Polyethylenterephthalatfolie von 25 µm Dicke wurde mit dieser Lösung beschichtet, so daß sich eine Trockenschichtdicke von 16 µm ergab. Die Oberfläche des trockenen Resistfilms wurde mit einer weiteren Polyethylenterephthalatfolie mit geringerer Haftung kaschiert. Der Trockenfilm wurde nach Abziehen der Deckfolie unter Druck und Wärme auf ein Messingblech laminiert. Nach Abkühlen und Abziehen der Trägerfolie wurde das Blech durch eine Vorlage hindurch belichtet, wobei ein guter Bildkontrast sichtbar wurde. Die belichteten Stellen wurden nach einer 20minütigen Lagerung mit einem Entwickler der in Beispiel 11 angegebenen Zusammensetzung sprühentwickelt. Das Blech wurde anschließend zu glatten Flanken durchgeätzt. Die erhaltenen Formteile können vor dem Vereinzeln noch weiter bearbeitet werden.

### Beispiel 30

Es wurde eine Beschichtungslösung hergestellt aus

| | |
|---|---|
| 7,5 Gt | des in Beispiel 9 angegebenen Novolaks, |
| 2,0 Gt | der Verbindung 14, |
| 0,05 Gt | Kristallviolettbase und |
| 0,6 Gt | 2-(4-Methoxy-styryl)-4,6-bis-trichlormethyl-1,3,5-triazin in |
| 42 Gt | Propylenglykol-monomethylether-acetat. |

Die Lösung wurde auf eine elektrolytisch aufgerauhte und anodisierte Aluminiumplatte, die zuvor mit einer wäßrigen Lösung von Polyvinylphosphonsäure behandelt worden war, aufgeschleudert und getrocknet. Das Schichtgewicht der getrockneten Schicht betrug 1,5 µm.

Die bildmäßige Belichtung erfolgte unter einer 5kW-Metallhalogenidlampe in 110 cm Abstand. Nach einer Wartezeit von 10 Minuten wurde die Platte mit dem in Beispiel 9 beschriebenen Entwickler entwickelt, wobei die belichteten Bereiche abgelöst wurden. Die so erhaltene Druckform wurde gummiert und in eine Flachdruckpresse eingespannt, wo sie mehr als 125.000 Drucke hervorragender Qualität lieferte.

## Patentansprüche

1. Verbindungen der allgemeinen Formel I 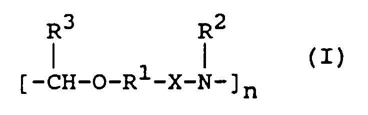 worin
R¹ eine Alkylen-, Cycloalkylen-, Alkenylen-, Alkinylen- oder Arylenbisalkylgruppe, in der eine oder mehrere aliphatische CH₂-Gruppe(n) durch Sauerstoff- oder Schwefelatome ersetzt sein können,
R² einen Alkyl-, Alkenyl-, Alkinyl-, Cycloalkyl-, Alkoxyalkyl-, Aryl-, Aralkyl- oder Aryloxyalkylrest,
R³ einen Alkyl- oder Arylrest,
X eine der Gruppen -CO-, -O-CO- oder -NH-CO- und
n eine ganze Zahl größer als 1
bedeuten.

2. Verbindungen nach Anspruch 1, dadurch gekennzeichnet, daß R¹ eine Alkylen-, Alkenylen- oder Alkinylengruppe mit 2 bis 18 Kohlenstoffatomen, in der 1 bis 3 CH₂-Gruppen durch Sauerstoff- oder Schwefelatome ersetzt sein können, eine Cycloalkylengruppe mit 4 bis 18 Kohlenstoffatomen, eine Aralkylengruppe mit 7 bis 18 Kohlenstoffatomen oder eine Arylenbisalkylgruppe mit 8 bis 18 Kohlenstoffatomen, bedeutet.

3. Verbindungen nach Anspruch 1, dadurch gekennzeichnet, daß R² einen Alkylrest mit 1 bis 12 Kohlenstoffatomen, einen Alkenyl- oder Alkinylrest mit 2 bis 12 Kohlenstoffatomen, einen Alkoxyalkylrest mit 3 bis 12 Kohlenstoffatomen, einen Cycloalkylrest mit 4 bis 12 Kohlenstoffatomen, einen Arylrest mit 6 bis 12 Kohlenstoffatomen, einen Aralkylrest mit 7 bis 12 Kohlenstoffatomen oder einen Aryloxyalkylrest mit 8 bis 12 Kohlenstoffatomen bedeutet.

4. Verbindungen nach Anspruch 1, dadurch gekennzeichnet, daß n eine ganze Zahl von 3 bis 50 bedeutet.

5. Strahlungsempfindliches Gemisch, das als wesentliche Bestandteile
(a) ein in Wasser unlösliches, in wäßrig-alka-lischen Lösungen lösliches oder zumindest quellbares Bindemittel,
(b) eine Verbindung, die unter Einwirkung von aktinischer Strahlung eine starke Säure bildet, und
(c) eine Verbindung enthält, die mindestens eine durch Säure spaltbare C-O-C-Bindung aufweist und deren Säurespaltprodukte sich in wäßrig-alkalischen Lösungen leichter lösen als die Verbindung selbst,
dadurch gekennzeichnet, daß die durch Säure spaltbare Verbindung (c) eine Verbindung gemäß einem der Ansprüche 1 bis 4 ist.

6. Strahlungsempfindliches Gemisch nach Anspruch 5, dadurch gekennzeichnet, daß die Verbindung (b) gegenüber Licht einer Wellenlänge von 150 bis 550 nm empfindlich ist.

7. Strahlungsempfindliches Gemisch nach Anspruch 5, dadurch gekennzeichnet, daß es die Verbindung (c) in einer Konzentration von 1 bis 60 Gew.-% enthält.

8. Strahlungsempfindliches Gemisch nach Anspruch 5, dadurch gekennzeichnet, daß das Bindemittel (a) im Wellenlängenbereich der Empfindlichkeit der Verbindung (b) eine Extinktion von weniger als 0,5 µm⁻¹ aufweist.

9. Strahlungsempfindliches Gemisch nach Anspruch 8, dadurch gekennzeichnet, daß das Bindemittel ein Polymeres mit phenolischen Hydroxylgruppen ist.

10. Strahlungsempfindliches Gemisch nach Anspruch 8, dadurch gekennzeichnet, daß das Bindemittel ein Gemisch verschiedener Polymere mit phenolischen Hydroxylgruppen enthält.

11. Strahlungsempfindliches Gemisch nach Anspruch 5, dadurch gekennzeichnet, daß es das Bindemittel (a) in einer Konzentration von 30 bis 95 Gew.-% enthält.

12. Strahlungsempfindliches Gemisch nach Anspruch 5, dadurch gekennzeichnet, daß es die Verbindungen (b) in einer Konzentration von 0,2 bis 25 Gew.-% enthält.

13. Strahlungsempfindliches Gemisch nach Anspruch 5, dadurch gekennzeichnet, daß die Verbindung (b) bei der Photolyse eine Sulfonsäure bildet.

14. Strahlungsempfindliches Aufzeichnungsmaterial aus einem Schichtträger und einer strahlungsempfindlichen Schicht, dadurch gekennzeichnet, daß die Schicht aus einem strahlungsempfindlichen Gemisch gemäß einem der Ansprüche 5 bis 13 besteht.

## Claims

1. A compound of the formula I 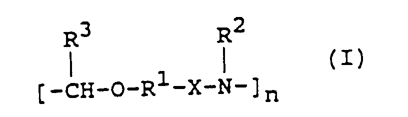 in which
R¹ is an alkylene, cycloalkylene, alkenylene, alkynylene or arylenebisalkyl group, in which one or more aliphatic CH₂ group(s) can have been replaced by oxygen or sulfur atoms,
R² is an alkyl, alkenyl, alkynyl, cycloalkyl, alkoxyalkyl, aryl, aralkyl or aryloxyalkyl radical,
R³ is an alkyl or aryl radical,
X is one of the groups -CO-, -O-CO- or -NH-CO- and
n is an integer greater than 1.

2. A compound as claimed in claim 1, wherein R¹ is an alkylene, alkenylene or alkynylene group having 2 to 18 carbon atoms, in which 1 to 3 CH₂ groups can have been replaced by oxygen or sulfur atoms, a cycloalkylene group having 4 to 18 carbon atoms, an aralkylene group having 7 to 18 carbon atoms or an arylenebisalkyl group having 8 to 18 carbon atoms.

3. A compound as claimed in claim 1, wherein R² is an alkyl radical having 1 to 12 carbon atoms, an alkenyl or alkynyl radical having 2 to 12 carbon atoms, an alkoxyalkyl radical having 3 to 12 carbon atoms, a cycloalkyl radical having 4 to 12 carbon atoms, an aryl radical having 6 to 12 carbon atoms, an aralkyl radical having 7 to 12 carbon atoms or an aryloxyalkyl radical having 8 to 12 carbon atoms.

4. A compound as claimed in claim 1, wherein n is an integer from 3 to 50.

5. A radiation-sensitive mixture which contains, as essential constituents,
(a) a binder which is insoluble in water and soluble or at least swellable in aqueous alkaline solutions,
(b) a compound which generates a strong acid under the action of actinic radiation and
(c) a compound which has at least one acid-cleavable C-O-C bond and whose acid cleavage products are more readily soluble in aqueous alkaline solutions than the compound itself,
wherein the acid-cleavable compound (c) is a compound as claimed in any one of claims 1 to 4.

6. A radiation-sensitive mixture as claimed in claim 5, wherein the compound (b) is sensitive to light of a wavelength from 150 to 550 nm.

7. A radiation-sensitive mixture as claimed in claim 5, which contains the compound (c) in a concentration from 1 to 60% by weight.

8. A radiation-sensitive mixture as claimed in claim 5, wherein the binder (a) has an extinction of less than 0.5 µm⁻¹ in the wavelength region of the sensitivity of the compound (b).

9. A radiation-sensitive mixture as claimed in claim 8, wherein the binder is a polymer having phenolic hydroxy groups.

10. A radiation-sensitive mixture as claimed in claim 8, wherein the binder contains a mixture of different polymers having phenolic hydroxy groups.

11. A radiation-sensitive mixture as claimed in claim 5, which contains the binder (a) in a concentration from 30 to 95% by weight.

12. A radiation-sensitive mixture as claimed in claim 5, which contains the compounds (b) in a concentration from 0.2 to 25% by weight.

13. A radiation-sensitive mixture as claimed in claim 5, wherein the compound (b) generates a sulfonic acid on photolysis.

14. A radiation-sensitive recording material comprising a layer support and a radiation-sensitive layer, wherein the layer is composed of a radiation-sensitive mixture as claimed in any one of claims 5 to 13.

## Revendications

1. Composés de formule générale I 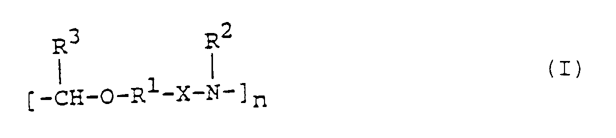 dans laquelle
R¹ représente un groupe alkylène, cycloalkylène, alcénylène, alcynylène ou arylènebisalkyle, dans lequel un ou plusieurs groupe(s) aliphatique(s) CH₂ peuvent être remplacés par des atomes d'oxygène ou de soufre,
R² représente un reste alkyle, alcényle, alcynyle, cycloalkyle, alcoxyalkyle, aryle, aralkyle ou aryloxyalkyle,
R³ représente un reste alkyle ou aryle,
X représente un des groupes -CO-, -O-CO- ou -NH-CO- et
n est un nombre entier supérieur à 1.

2. Composés selon la revendication 1, caractérisés en ce que R¹ représente un groupe alkylène, alcénylène ou alcynylène avec 2 à 18 atomes de carbone, dans lequel 1 à 3 groupes CH₂ peuvent être remplacés par des atomes d'oxygène ou de soufre, un groupe cycloalkylène avec 4 à 18 atomes de carbone, un groupe aralkylène avec 7 à 18 atomes de carbone ou un groupe arylènebisalkyle avec 8 à 18 atomes de carbone.

3. Composés selon la revendication 1, caractérisés en ce que R² représente un reste alkyle avec 1 à 12 atomes de carbone, un reste alcényle ou alcynyle avec 2 à 12 atomes de carbone, un reste alcoxyalkyle avec 3 à 12 atomes de carbone, un reste cycloalkyle avec 4 à 12 atomes de carbone, un reste aryle avec 6 à 12 atomes de carbone, un reste aralkyle avec 7 à 12 atomes de carbone ou un reste aryloxyalkyle avec 8 à 12 atomes de carbone.

4. Composés selon la revendication 1, caractérisés en ce que n est un nombre entier de 3 à 50.

5. Mélange sensible au rayonnement contenant comme constituants essentiels
(a) un liant insoluble dans l'eau, soluble ou au moins gonflable dans des solutions aqueuses alcalines,
(b) un composé qui forme un acide fort sous l'effet d'un rayonnement actinique, ainsi que
(c) un composé qui présente au moins une liaison C-O-C que l'on peut cliver par un acide, et que l'on peut transformer par clivage en un produit avec une solubilité accrue dans des solutions aqueuses alcalines,
caractérisé en ce que le composé (c) que l'on peut cliver par un acide est un composé selon l'une des revendications 1 à 4.

6. Mélange sensible au rayonnement selon la revendication 5, caractérisé en ce que le composé (b) est sensible à la lumière d'une longueur d'onde de 150 à 550 nm.

7. Mélange sensible au rayonnement selon la revendication 5, caractérisé en ce qu'il contient le composé (c) dans une concentration de 1 à 60 % en poids.

8. Mélange sensible au rayonnement selon la revendication 5, caractérisé en ce que le liant (a) présente dans le domaine des longueurs d'onde de la sensibilité du composé (b), une extinction inférieure à 0,5 µm⁻¹.

9. Mélange sensible au rayonnement selon la revendication 8, caractérisé en ce que le liant est un polymère comportant des groupes hydroxyle phénoliques.

10. Mélange sensible au rayonnement selon la revendication 8, caractérisé en ce que le liant contient un mélange de différents polymères avec des groupes hydroxyle phénoliques.

11. Mélange sensible au rayonnement selon la revendication 5, caractérisé en ce qu'il contient le liant (a) dans une concentration de 30 à 95 % en poids.

12. Mélange sensible au rayonnement selon la revendication 5, caractérisé en ce qu'il contient les composés (b) dans une concentration de 0,2 à 25 % en poids.

13. Mélange sensible au rayonnement selon la revendication 5, caractérisé en ce que le composé (b) forme un acide sulfonique au cours de la photolyse.

14. Matière d'enregistrement sensible au rayonnement constituée d'un support de couche et d'une couche sensible au rayonnement, caractérisée en ce que la couche est constituée d'un mélange sensible au rayonnement selon l'une des revendications 5 à 13.
